# EUROPÄISCHE PATENTSCHRIFT

(11) **EP 3 956 286 B1**
(45) Veröffentlichungstag und Bekanntmachung des Hinweises auf die Patenterteilung: **13.12.2023**
(21) Anmeldenummer: 19730330.8
(22) Anmeldetag: 12.06.2019
(51) Int. Cl.: C07C 67/31, C07C 69/675, A61K 31/22, A23L 33/00, A61P 3/00, A61P 25/00

(54) **VERFAHREN ZUR HERSTELLUNG VON POLYOLBASIERTEN ESTERN, INSBESONDERE POLYGLYCERINESTERN, VON HYDROXYCARBONSÄUREN**
METHOD FOR PRODUCING POLYOL-BASED ESTERS, IN PARTICULAR POLYGLYCEROL ESTERS, FROM HYDROXY CARBOXYLIC ACIDS
PROCÉDÉ DE PRÉPARATION D'ESTERS À BASE POLYOL, NOTAMMENT D'ESTERS DE POLYGLYCÉROL, D'ACIDES HYDROXYCARBOXYLIQUES

(43) Veröffentlichungstag der Anmeldung: 23.02.2022
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/065271
(87) Internationale Veröffentlichungsnummer: WO 2020/249196

(56) Entgegenhaltungen:
- WO-A1-95/09144
- WO-A1-99/02549
- WO-A1-2010/021766
- WO-A1-2013/150153
- WO-A1-2018/132189
- WO-A2-2004/108740

## Beschreibung

Die vorliegende Erfindung ist in den Ansprüchen definiert und betrifft das Gebiet der Ketokörper und des damit zusammenhängenden Stoffwechsels sowie die Therapie von damit im Zusammenhang stehenden Erkrankungen. Die vorliegende Erfindung ist ein Verfahren zur Herstellung von gegebenenfalls funktionalisierten, vorzugsweise gegebenenfalls fettsäurefunktionalisierten, bevorzugt gegebenenfalls C₅-C₃₄-fettsäurefunktionalisierten, insbesondere gegebenenfalls C₈-C₃₄-fettsäurefunktionalisierten, Polyolestern, insbesondere Polyglycerinestern, von 3-Hydroxybuttersäure sowie die auf diese Weise erhältlichen bzw. hergestellten Reaktionsprodukte (d. h. gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, Polyolester, insbesondere Polyglycerinester, von 3-Hydroxybuttersäure, bevorzugt in enantiomerenangereicherter bzw. enantiomerenreiner Form) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, Polyolester, insbesondere Polyglycerinester, von 3-Hydroxybuttersäure, bevorzugt in enantiomerenangereicherter bzw. enantiomerenreiner Form) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*), *Novel Food*, Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. gegebenenfalls funktionalisierte, vorzugsweise gegebenenfalls fettsäurefunktionalisierte, bevorzugt gegebenenfalls C₅-C₃₄-fettsäurefunktionalisierten, insbesondere gegebenenfalls C₈-C₃₄-fettsäurefunktionalisierten, Polyolester, insbesondere Polyglycerinester, von 3-Hydroxybuttersäure, bevorzugt in enantiomerenangereicherter bzw. enantiomerenreiner Form) umfassen, sowie deren Anwendungen bzw. Verwendungen.

Der Begriff des gegebenenfalls funktionalisierten, vorzugsweise gegebenenfalls fettsäurefunktionalisierten, bevorzugt gegebenenfalls C₅-C₃₄-fettsäurefunktionalisierten, insbesondere gegebenenfalls C₈-C₃₄-fettsäurefunktionalisierten, Polyolesters, insbesondere Polyglycerinesters, von 3-Hydroxybuttersäure, wie er erfindungsgemäß verwendet wird, bezeichnet insbesondere Verbindungen, deren Hydroxylgruppen gegebenenfalls funktionalisiert, insbesondere fettsäurefunktionalisiert, bevorzugt C₅-C₃₄-fettsäurefunktionalisiert, besonders bevorzugt C₈-C₃₄-fettsäurefunktionalisiert, sind (d. h. das Wasserstoffatom des oder der Hydroxylgruppen des Polyols und/oder der 3-Hydroxybuttersäure ist gegebenenfalls durch eine Acylgruppe, d. h. also eine Gruppe - CH(OR) - mit R = Acyl, insbesondere R = (C₅-C₃₄-Alkyl) - C(O) -, vorzugsweise R = (C₈-C₃₄-Alkyl) - C(O) -, ersetzt, sodass eine Estergruppe vorliegt). Folglich können die erfindungsgemäßen Reaktionsprodukte (d. h. gegebenenfalls funktionalisierte, vorzugsweise gegebenenfalls fettsäurefunktionalisierte, bevorzugt gegebenenfalls C₅-C₃₄-fettsäurefunktionalisierte, insbesondere gegebenenfalls C₈-C₃₄-fettsäurefunktionalisierte, Polyolester, insbesondere Polyglycerinester, von 3-Hydroxybuttersäure) gegebenenfalls eine Acylierung an freien Hydroxylgruppen des Polyols und/oder der 3-Hydroxybuttersäure bzw. des 3-Hydroxybutyrats (d. h. Acylierung in der 3-Position des 3-Hydroxybutyratrests im betreffenden Ester) aufweisen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glykogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper oder Englisch auch als "*Keton Bodies*" bezeichnet).

Der Begriff der Ketokörper ist insbesondere eine Sammelbezeichnung für Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter dem Begriff der Ketokörper fasst man insbesondere die drei Verbindungen Acetoacetat (synonym auch Acetacetat oder 3-Oxobutyrat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2004/108740 A2 betrifft Verbindungen und Zusammensetzungen, welche (R)-3-Hydroxybutyrat-Derivate enthalten, wobei die Verbindungen und Zusammensetzungen als Nahrungsergänzungsmittel zur Steigerung der körperlichen Leistungsfähigkeit und als Therapeutikum zur Linderung der Symptome von Erkrankungen, insbesondere neurologischen Erkrankungen wie Alzheimer oder ähnlichen Erkrankungen, verwendet werden, sowie deren Herstellungsverfahren, wobei in dem Verfahren beispielsweise ein überkritisches Lösemittel wie zum Beispiel überkritisches Kohlendioxid verwendet wird und eine Lipase-katalysierte Veresterungs- oder Umesterungsreaktion durchgeführt wird, um die (R)-3-Hydroxybutyrat-Derivate herzustellen.

Darüber hinaus betrifft die WO 95/09144 A1 Zusammensetzungen, welche als parenterale Nährstoffe nützlich sind, wobei diese Verbindungen wasserlösliche Glycerinester der 3-Hydroxybuttersäure sind, wobei die Zusammensetzung als Ersatz für Glykose bei der intravenösen Nahrungszufuhr nützlich sein sollen.

Weiterhin betrifft die WO 2018/132189 A1 ein Verfahren zur Behandlung von leichten bis mittelschweren geschlossenen traumatischen Hirnverletzungen (TBI) und leichten bis mittelschwerer TBI aufgrund eines chirurgischen Eingriffs unter Verwendung von 3-Hydroxybutyratglyceriden.

Des Weiteren betrifft die WO 2010/021766 A1 eine Verbindung, welche in Bezug auf (3R)-Hydroxybutyl-(3R)-Hydroxybutyrat ein enantiomer angereichertes 3-Hydroxybutyl-3-Hydroxybutyrat der Formel (I) ist, wobei die Verbindungen der Formel (I) ein wirksamer und schmackhafter Vorläufer des Ketokörpers (3R)-Hydroxybutyrat sind und zur Behandlung eines Zustands verwendet werden können, welcher durch einen erhöhten Plasmaspiegel an freien Fettsäuren bei einem Menschen oder Tier verursacht, verschlimmert oder damit verbunden ist (beispielsweise eine Zustand, bei welchem Gewichtsverlust oder Gewichtszunahme eine Rolle spielt) oder aber zur Förderung der Wachsamkeit oder zur Verbesserung der kognitiven Funktion oder zur Behandlung, Verhinderung oder Verringerung der Auswirkungen von Neurodegeneration, Toxizität durch freie Radikale, hypoxische Zustände oder Hyperglykämie eingesetzt werden können.

Die WO 2013/150153 A1 betrifft Ketokörper und Ketokörperester zur oralen Verabreichung zur Verbesserung oder zum Erhalt der Muskelkraft, wobei bestimmte Ester von Hydroxybutyratmonomeren organoleptisch akzeptabel sind und eine hohe Aufnahme vom Darm ins Blut aufweisen sollen, wodurch ein schneller Anstieg der Hydroxybutyratkonzentration im Blut und eine physiologische Reaktion einschließlich einer verbesserten Leistungsabgabe während des Trainings ermöglicht werden soll, sowie Zusammensetzungen, welche diese Ketokörper oder Ketokörperester enthalten.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass gegebenenfalls C₅-C₃₄-fettsäurefunktionalisierte, insbesondere gegebenenfalls C₈-C₃₄-fettsäurefunktionalisierte, Polyglycerinester von 3-Hydroxybuttersäure (beta-Hydroxybuttersäure, BHB bzw. 3-BHB) einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von gegebenenfalls C₅-C₃₄-fettsäurefunktionalisierten, insbesondere gegebenenfalls C₈-C₃₄-fettsäurefunktionalisierten, Polyglycerinestern von 3-Hydroxybuttersäure (beta-Hydroxybuttersäure, BHB bzw. 3-BHB) gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt gemäß dem diesbezüglichen Anspruch (Anspruch 8) bzw. einen gegebenenfalls C₅-C₃₄-fettsäurefunktionalisierten, insbesondere gegebenenfalls C₈-C₃₄-fettsäurefunktionalisierten, Polyglycerinester von 3-Hydroxybuttersäure (beta-Hydroxybuttersäure, BHB bzw. 3-BHB) gemäß den diesbezüglichen Ansprüchen (Ansprüche 9 bis 11) bzw. ein diesbezüglich erhältliches Gemisch von mindestens zwei, vorzugsweise mindestens drei, voneinander verschiedene, gegebenenfalls C₅-C₃₄-fettsäurefunktionalisierten, insbesondere gegebenenfalls C₈-C₃₄-fettsäurefunktionalisierten, Polyglycerinestern von 3-Hydroxybuttersäure (beta-Hydroxybuttersäure, BHB bzw. 3-BHB), gemäß dem diesbezüglichen Anspruch (Anspruch 12); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 13); weitere, insbesondere besondere und/oder vorteilhafte Ausgestaltungen dieses Erfindungsaspekts sind Gegenstand des diesbezüglichen Unteranspruchs.

Des Weiteren betrifft die vorliegende Erfindung - gemäß einem **vierten** Aspekt der vorliegenden Erfindung - ein Nahrungsmittel und/oder Lebensmittelerzeugnis gemäß dem diesbezüglichen unabhängigen Anspruch (Anspruch 15.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung eines gegebenenfalls C₅-C₃₄-fettsäurefunktionalisierten, insbesondere gegebenenfalls C₈-C₃₄-fettsäurefunktionalisierten, Polyglycerinesters von 3-Hydroxybuttersäure,
wobei ein gegebenenfalls C₅-C₃₄-fettsäurefunktionalisierter, insbesondere gegebenenfalls C₈-C₃₄-fettsäurefunktionalisierter, 3-Oxobuttersäure-Polyglycerinester (I), welcher mindestens einen 3-Oxobutyratrest (3-Oxobuttersäurerest) der allgemeinen Formel (I')

   CH₃ - C(O) - CH₂ - C(O)O - (I')
aufweist, mittels mindestens eines Reduktionsmittels einer selektiven Reduktion des mindestens einen 3-Oxobutyratrests der allgemeinen Formel (I') an der Ketogruppe - C(O) - der Acetylfunktion CH₃ - C(O) - zu einem 3-Hydroxybutyratrest (3-Hydroxybuttersäurerest) der allgemeinen Formel (II')

   CH₃ - CH(OH) - CH₂ - C(O)O - (II')
unterzogen wird,
wobei der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) der allgemeinen Formel (Ib)

   R¹O - CH₂ - CH(OR¹) - CH₂ - [O - CH₂ - CH(OR¹) - CH₂]ₚ - OR¹ (Ib)
entspricht,
wobei in der allgemeinen Formel (Ib)
   - die Variable p eine ganze Zahl von 1 bis 4, vorzugsweise 1 oder 2, besonders bevorzugt 1, darstellt,
   - der Rest R¹ jeweils unabhängig voneinander, gleich oder verschieden, darstellt: Wasserstoff, CH₃ - C(O) - CH₂ - C(O) - oder einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, jedoch mit der Maßgabe, dass mindestens ein Rest R¹, insbesondere mindestens zwei Reste R¹, keinen Wasserstoff darstellt, und mit der Maßgabe, dass mindestens ein Rest R¹, insbesondere mindestens zwei Reste R¹, einen Rest CH₃- C(O) - CH₂ - C(O) - darstellt; und
wobei das Reduktionsmittel ausgewählt ist aus der Gruppe von Wasserstoff, Hydriden und Alkoholen sowie deren Mischungen; und
wobei die Reduktion als eine vollständige Reduktion oder aber als nicht-vollständige Reduktion sämtlicher Ketogruppen - C(O) - der Acetylfunktion CH₃ - C(O) - des mindestens einen 3-Oxobutyratrests der allgemeinen Formel (I') zu einem 3-Hydroxybutyratrest der allgemeinen Formel (II') durchgeführt wird;
so dass als Reaktionsprodukt ein gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester (II) erhalten wird.

Wie zuvor ausgeführt, hat die Anmelderin nämlich vollkommen überraschend herausgefunden, dass die auf diese Weise hergestellten gegebenenfalls C₅-C₃₄-fettsäurefunktionalisierten, insbesondere gegebenenfalls C₈-C₃₄-fettsäurefunktionalisierten, Polyglycerinester von 3-Hydroxybuttersäure (beta-Hydroxybuttersäure, BHB bzw. 3-BHB) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten gegebenenfalls funktionalisierten Polyglycerinester von 3-Hydroxybuttersäure, welche durch das erfindungsgemäße Herstellungsverfahren in effizienter Weise zugänglich sind, stellen eine physiologisch und pharmakologisch relevante Alternative zu der freien 3-Hydroxybuttersäure bzw. deren Salzen dar.

Die Herstellung von gegebenenfalls funktionalisierten Polyglycerinestern von 3-Hydroxybuttersäure mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte ein effizient arbeitendes Herstellungsverfahren bereitgestellt werden, mit welchem sich gegebenenfalls funktionalisierte Polyglycerinester von 3-Hydroxybuttersäure ohne unerwünschte Nebenreaktionen herstellen lassen.

Das erfindungsgemäße Verfahren ermöglicht somit die Bereitstellung untoxischer gegebenenfalls funktionalisierter Polyglycerinester von 3-Hydroxybuttersäure aus physiologisch unbedenklichen Komponenten bzw. Edukten (Ausgangsverbindungen). Die resultierenden gegebenenfalls funktionalisierten Polyglycerinester von 3-Hydroxybuttersäure können physiologisch, insbesondere im Magen und/oder im Darm aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten gegebenenfalls funktionalisierten Polyglycerinester von 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die gegebenenfalls funktionalisierten Polyglycerinester von 3-Hydroxybuttersäure frei von toxischen Verunreinigungen bereitzustellen.

Darüber hinaus kann die Herstellung auch enantioselektiv, insbesondere mittels chiraler Katalyse, durchgeführt werden. So ermöglicht es beispielsweise das erfindungsgemäße Herstellungsverfahren, die biologisch relevante Form, d. h. das (R)-Enantiomer, anzureichern bzw. ausschließlich herzustellen, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern bzw. ausschließlich herzustellen.

Darüber hinaus ist das erfindungsgemäße Herstellungsverfahren einschließlich optionaler Weiterverarbeitungs- bzw. Aufreinigungsverfahrensschritte wirtschaftlich bzw. ökonomisch betreibbar und auch großtechnisch umsetzbar.

Insbesondere verwendet das erfindungsgemäße Herstellungsverfahren einfach zugängliche Edukte und ermöglicht darüber hinaus eine relativ einfache Verfahrensführung auch bei großtechnischer Umsetzung. Dennoch werden im Rahmen des erfindungsgemäßen Herstellungsverfahrens exzellente Ausbeuten erzielt, wobei in gleicher Weise die Bildung von Nebenprodukten minimiert (keine signifikanten Mengen an Nebenprodukten) bzw. vermieden wird.

Im Gegensatz zu herkömmlichen Herstellungsverfahrens des Standes der Technik sind auch die Edukte physiologisch kompatibel und sogar pharmazeutisch wirksam, d. h. gegebenenfalls nicht umgesetztes Edukt kann im Endprodukt verbleiben, wodurch keine bzw. kaum Aufreinigungsschritte nötig sind (auch wenn eine Entfernung ohne Weiteres möglich ist, sofern gewünscht). Insbesondere wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Darüber hinaus verwendet das erfindungsgemäße Herstellungsverfahren kommerziell verfügbare, nicht toxische und pharmakologisch kompatible Reduktionsmittel.

Gemäß einer besonderen Ausführungsform führt das erfindungsgemäße Herstellungsverfahren typischerweise zu einem Gemisch verschiedener gegebenenfalls funktionalisierter Polyglycerinester von 3-Hydroxybuttersäure, d. h. zu einem Gemisch von mindestens zwei, insbesondere mindestens drei voneinander verschiedenen gegebenenfalls funktionalisierten Polyglycerinestern von 3-Hydroxybuttersäure. Das resultierende Reaktionsprodukt bzw. Rohgemisch kann bei Bedarf mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen gegebenenfalls funktionalisierten Polyolester, d. h. Mono-, Di-, Triusw. gegebenenfalls funktionalisierten Polyolester von 3-Hydroxybuttersäure, oder aber Fraktionierung in Fraktionen mit angereichertem und abgereichertem Anteil einzelner gegebenenfalls funktionalisierter Polyolester etc.).

Mit dem erfindungsgemäßen Herstellungsverfahren lassen sich erforderlichenfalls jedoch auch die reinen bzw. einzelnen gegebenenfalls funktionalisierten Polyglycerinester von 3-Hydroxybuttersäure als Rein- bzw. Einzelstoffe herstellen (z. B. in Abhängigkeit von den eingesetzten Edukten).

Das erfindungsgemäße Herstellungsverfahren ermöglicht weiterhin eine gezielte partielle Reduktion (Hydrolyse) durch die Steuerung der Reaktion, insbesondere der Menge an Reduktionsmittel; mit anderen Worten kann erforderlichenfalls auch nur ein gewisser, insbesondere definierter Anteil der 3-Oxobutyratreste bzw. der darin enthaltenen Ketogruppen - C(O) -zu einem 3-Hydroxybutyratrest bzw. der entsprechenden Hydroxylfunktion -CH(OH) - umgesetzt werden. Hierdurch ist es möglich, ein Reaktionsprodukt zur Verfügung zu stellen, welches einen weiterführenden Retard-Effekt aufweist. Durch die Anwesenheit von sowohl 3-Oxobutyratresten und 3-Hydroxybutyratresten in einem entsprechenden Produktgemisch liegt eine unterschiedlich schnelle Verfügbarkeit bzw. Freisetzung des Wirkstoffs 3-Hydroxybuttersäure vor.

Im Rahmen des erfindungsgemäßen Herstellungsverfahrens erfolgt eine selektive Reduktion nur der Ketogruppe - C(O) - der Acetylfunktion CH₃ - C(O) - zu einer Hydroxylgruppe - CH(OH) -, d. h. es finden keine Nebenreaktionen, insbesondere keine Umlagerungen, Abspaltungen, Additionen etc., statt.

Im Gegensatz zu herkömmlichen Herstellungsverfahren des Standes der Technik kommt es bei dem erfindungsgemäßen Herstellungsverfahren nicht zu einer Dimerisierung der 3-Hydroxybuttersäure, wie dies z. B. im Fall der direkten Umsetzung von 3-Hydroxybuttersäure mit einem Polyolester erfolgt.

Insgesamt ist das erfindungsgemäße Verfahren somit sowohl einfach als auch wirtschaftlich bzw. ökonomisch und somit auch großtechnisch betreibbar.

Mit anderen Worten betrifft die vorliegende Erfindung somit ein Verfahren zur Herstellung eines gegebenenfalls C₅-C₃₄-fettsäurefunktionalisierten, insbesondere gegebenenfalls C₈-C₃₄-fettsäurefunktionalisierten, Polyglycerinesters von 3-Hydroxybuttersäure (beta-Hydroxybuttersäure, BHB bzw. 3-BHB),
wobei ein gegebenenfalls C₅-C₃₄-fettsäurefunktionalisierter, insbesondere gegebenenfalls C₈-C₃₄-fettsäurefunktionalisierter, 3- 3-Oxobuttersäure-Polyglycerinester (I) welcher mindestens einen 3-Oxobutyratrest (3-Oxobuttersäurerest) der allgemeinen Formel (I')

   CH₃ - C(O) - CH₂ - C(O)O - (I')
aufweist, einer selektiven Reduktion der Ketogruppe - C(O) - der Acetylfunktion CH₃ - C(O) - in dem oder den 3-Oxobutyratresten der allgemeinen Formel (I') zu einer Hydroxylfunktion - CH(OH) - mittels mindestens eines Reduktionsmittels unterzogen wird,
so dass als Reaktionsprodukt ein gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester(I) erhalten wird.

Im Rahmen der Erfindung ist unter dem Begriff der selektiven Reduktion insbesondere nur die Reduktion der Ketogruppe - C(O) - der Acetylfunktion CH₃ - C(O) - des 3-Oxobutyratrests der allgemeinen Formel (I'), wie zuvor definiert, zu einer Hydroxylgruppe - CH(OH) - des 3-Hydroxybutyratrests der allgemeinen Formel (II'), wie zuvor definiert, zu verstehen. Es finden insbesondere während dieser selektiven Reduktion keine Nebenreaktionen, insbesondere keine Umlagerungen, Abspaltungen, Additionen etc., statt und weiterhin wird auch keine weitere bzw. andere funktionelle Gruppe (d. h. keine funktionelle Gruppe außer der zuvor genannten Ketogruppe) reduziert.

Auch lässt sich im Rahmen einer chiralen Katalyse, wie nachfolgend noch im Detail ausgeführt, das Reaktionsprodukt in entiomerenangereicherter Form oder bevorzugt sogar in enantiomerenreiner Form (z. B. mindestens 95 %, insbesondere mindestens 99 % Enantiomerenreinheit) erhalten (z. B. als (R)-Enantiomer gemäß nachstehend erläuterter allgemeiner Formel (II')).

Das im Rahmen des erfindungsgemäßen Verfahrens einsetzbare Reduktionsmittel kann in weiten Bereichen variiert werden. Erfindungsgemäß ist das Reduktionsmittel ausgewählt aus der Gruppe von Wasserstoff, Hydriden, insbesondere anorganischen Hydriden, und Alkoholen, insbesondere C₁-C₄-Alkoholen, sowie deren Mischungen, vorzugsweise ausgewählt aus der Gruppe von Wasserstoff, Alkali- oder Erdalkaliborhydrid, Alkali- oder Erdalkalihydrid, Alkali- oder Erdalkalialuminiumhydrid, Methanol, Ethanol, Propanol und Isopropanol sowie deren Mischungen, bevorzugt aus der Gruppe von Wasserstoff, Alkali- oder Erdalkaliborhydrid und Isopropanol.

Im Rahmen der vorliegenden Erfindung kann die Reduktion autokatalytisch oder in Gegenwart eines Katalysators durchgeführt werden.

Insbesondere wird bei dem erfindungsgemäßen Verfahren im Fall der Durchführung der Reduktion in Gegenwart eines Katalysators, der Katalysator nach erfolgter Reduktion rezykliert.

Wie zuvor ausgeführt, kann gemäß einer besonderen Ausführungsform des erfindungsgemäßen Herstellungsverfahrens die Reduktion autokatalytisch durchgeführt werden.

Für den Fall, dass die Reduktion autokatalytisch durchgeführt wird, kann das Reduktionsmittel insbesondere ein Hydrid, vorzugsweise ein anorganisches Hydrid, bevorzugt Alkali- oder Erdalkaliborhydrid, Alkali- oder Erdalkalihydrid und/oder Alkali- oder Erdalkalialuminiumhydrid, besonders bevorzugt Alkali- oder Erdalkaliborhydrid, sein.

Sofern die Reduktion im Rahmen des erfindungsgemäßen Herstellungsverfahrens mit einem Hydrid durchgeführt wird, ist es bevorzugt, wenn die Reduktion bei Temperaturen im Bereich von 2 °C bis 30 °C, insbesondere im Bereich von 3 °C bis 25 °C, vorzugsweise im Bereich von 3 °C bis 20 °C, bevorzugt im Bereich von 3 °C bis 15 °C, besonders bevorzugt im Bereich von 3 °C bis 12 °C, durchgeführt wird.

Wenn gemäß einer besonderen Ausführungsform der vorliegenden Erfindung die Reduktion mit einem Hydrid durchgeführt wird, kann der angewendete Druckbereich in weiten Bereichen variieren. Insbesondere kann bei der Reduktion mit einem Hydrid die Reduktion bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Im Fall der Verwendung eines Hydrids als Reduktionsmittel kann die Menge des eingesetzten Hydrids in weiten Mengen variieren. Insbesondere kann das Hydrid in Mengen, bezogen auf die Menge des gegebenenfalls C₅-C₃₄-fettsäurefunktionalisierten, insbesondere gegebenenfalls C₈-C₃₄-fettsäurefunktionalisierten, 3-Oxobuttersäure-Polyglycerinesters (I) im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Gemäß einer alternativen Ausführungsform der vorliegenden Erfindung kann die Reduktion in Gegenwart eines Katalysators durchgeführt werden.

Wie zuvor bereits dargestellt, ist es bevorzugt, bei der Verwendung eines Katalysators diesen nach erfolgter Reduktion zu rezyklieren.

Sofern die Reduktion im Rahmen des erfindungsgemäßen Herstellungsverfahrens in Gegenwart eines Katalysators durchgeführt wird, ist es bevorzugt, wenn der Katalysator ausgewählt ist aus der Gruppe von Enzymen und/oder Metallen oder Metallverbindungen, insbesondere Edelmetallen und Übergangsmetallverbindungen.

In diesem Zusammenhang ist es besonders bevorzugt, wenn der Katalysator ausgewählt ist aus der Gruppe von Dehydrogenasen, insbesondere Alkoholdehydrogenasen, und Metallen und/oder Metallverbindungen auf Basis von Palladium, Platin, Rhodium, Iridium, Ruthenium und Nickel, vorzugsweise aus der Gruppe von Dehydrogenasen, insbesondere Alkoholdehydrogenasen, und Metallen und/oder Metallverbindungen auf Basis von Platin, Palladium, Nickel und Rhodium.

Bei Dehydrogenasen handelt es sich um Enzyme, die ihr Substrat durch Abspaltung von Wasserstoffanionen (d.h. H⁻) oxidieren. Dehydrogenasen sind folglich nicht zu verwechseln mit Dehydratasen, welche Wasser abspalten. Dehydrogenasen gehören zur Gruppe I (Oxidoreduktasen) der EC-Klassifikation der Enzymgruppen. Die Elektronen sowie der abgespaltene Wasserstoff werden auf Co-Faktoren, wie NAD⁺ oder FAD, übertragen. Abhängig vom Substrat können verschiedene Dehydrogenasen unterschieden werden; beispielsweise handelt es sich bei dem Enzym, welches in der Leber beim Alkoholabbau Ethanol in Acetaldehyd (Ethanal) umwandelt, um eine Alkoholdehydrogenase (EC 1.1.1.1). Alkoholdehydrogenasen (ADH) sind Enzyme, die sowohl die Reaktion von Alkoholen zu den entsprechenden Aldehyden oder Ketonen als auch die Rückreaktion (Aldehyd bzw. Keton zu Alkohol) dieser katalysieren. Bei dieser Reaktion handelt es sich um eine Redoxreaktion.

Erfindungsgemäß besonders geeignete Katalysatoren auf Basis von Metallen und/oder Metallverbindungen sind der Wilkinson-Katalysator, welcher ein Homogenkatalysator mit der Summenformel C₅₄H₄₅ClP₃Rh ist. Es handelt sich hierbei um einen Rhodiumkomplex, der bei der Hydrierung, Hydroformylierung, Hydrosilylierung und zur Isomerisierung Anwendung findet. Ein weiterer erfindungsgemäß besonders geeigneter Katalysator ist Raney-Nickel, welches ein fester Katalysator aus feinen Körnern einer Nickel/Aluminium-Legierung ist.

Wie zuvor ausgeführt, kann gemäß einer besonderen Ausführungsform des erfindungsgemäßen Herstellungsverfahrens die Reduktion in Gegenwart eines Enzyms als Katalysator durchgeführt werden.

Dabei kann das Enzym vorzugsweise eine Dehydrogenase, insbesondere eine Alkoholdehydrogenase, sein.

Wie zuvor im Zusammenhang mit der Verwendung eines Katalysators im Allgemeinen dargelegt, ist es bevorzugt, im Fall der Verwendung eines Enzyms als Katalysator das Enzym nach der Reduktion zu rezyklieren.

Sofern die Reduktion im Rahmen des erfindungsgemäßen Herstellungsverfahrens in Gegenwart eines Enzyms als Katalysator durchgeführt wird, ist es bevorzugt, wenn die Reduktion bei Temperaturen im Bereich von 5 °C bis 80 °C, insbesondere im Bereich von 10 °C bis 65 °C, vorzugsweise im Bereich von 10 °C bis 50 °C, bevorzugt im Bereich von 15 °C bis 40 °C, besonders bevorzugt im Bereich von 15 °C bis 30 °C, durchgeführt wird.

Im Rahmen dieser besonderen Ausführungsform der vorliegenden Erfindung, bei der die Reduktion in Gegenwart eines Enzyms als Katalysator durchgeführt wird, ist es bevorzugt, wenn die Reduktion bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

Dennoch kann es einzelfallbedingt oder anwendungsbezogen erforderlich sein, von den vorgenannten Temperatur- und Druckbereichen abzuweichen, ohne dass der Rahmen der vorliegenden Erfindung verlassen ist.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den genannten Mengen abzuweichen, ohne dass der Rahmen der vorliegenden Erfindung verlassen ist. In diesem Zusammenhang sind unter Ausgangsverbindungen der gegebenenfalls C₅-C₃₄-fettsäurefunktionalisierte, insbesondere gegebenenfalls C₈-C₃₄-fettsäurefunktionalisierte, 3-Oxobuttersäure-Polyolester (I) und das Reduktionsmittel zu verstehen.

Gemäß einer alternativen Ausführungsform des erfindungsgemäßen Verfahrens kann die Reduktion in Gegenwart eines Metalls und/oder einer Metallverbindung als Katalysator durchgeführt werden.

In diesem Zusammenhang ist es bevorzugt, wenn das Metall und/oder die Metallverbindung ausgewählt ist aus Edelmetallen und Übergangsmetallverbindungen.

Wie zuvor im Zusammenhang mit der Verwendung eines Katalysators im Allgemeinen dargelegt, ist es bevorzugt, im Fall der Verwendung eines Metalls und/oder einer Metallverbindung als Katalysator das Metall und/oder die Metallverbindung nach erfolgter Reduktion zu rezyklieren.

Gemäß dieser besonderen Ausführungsform kann der Katalysator ausgewählt sein aus der Gruppe von Metallen und/oder Metallverbindungen auf Basis von Palladium, Platin, Rhodium, Iridium, Ruthenium und Nickel, vorzugsweise aus der Gruppe von Metallen und/oder Metallverbindungen auf Basis von Platin, Palladium, Nickel und Rhodium.

Sofern die Reduktion im Rahmen des erfindungsgemäßen Verfahrens in Gegenwart eines Metalls als Katalysator durchgeführt wird, kann der angewendete Temperaturbereich in weiten Bereichen variieren. Insbesondere kann die Reduktion in Gegenwart eines Metalls als Katalysator bei Temperaturen im Bereich von 10 °C bis 140 °C, insbesondere im Bereich von 15 °C bis 135 °C, vorzugsweise im Bereich von 20 °C bis 130 °C, bevorzugt im Bereich von 25 °C bis 125 °C, besonders bevorzugt im Bereich von 35 °C bis 120 °C, ganz besonders bevorzugt im Bereich von 40 °C bis 110 °C, durchgeführt werden.

Wenn gemäß dieser besonderen Ausführungsform der vorliegenden Erfindung die Reduktion in Gegenwart eines Metalls als Katalysator durchgeführt wird, kann auch der angewendete Druckbereich in weiten Bereichen variieren. Insbesondere kann bei der Reduktion in Gegenwart eines Metalls als Katalysator die Reduktion bei einem Druck im Bereich von 2 bar bis 80 bar, insbesondere im Bereich von 5 bar bis 70 bar, vorzugsweise im Bereich von 10 bar bis 60 bar, besonders bevorzugt im Bereich von 15 bar bis 55 bar, ganz besonders im Bereich von 20 bar 50 bar, durchgeführt werden.

Im Fall der Verwendung eines Metalls als Katalysator kann auch die Menge des eingesetzten Metalls in weiten Bereichen variieren. Insbesondere kann das Metall in Mengen, bezogen auf die Gesamtmenge der Ausgangsverbindungen, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden.

Dennoch kann es einzelfallbedingt oder anwendungsbezogen erforderlich sein, von den vorgenannten Mengen sowie Temperatur- und Druckbereichen abzuweichen, ohne dass der Rahmen der vorliegenden Erfindung verlassen ist.

Gemäß einer Ausführungsform der vorliegenden Erfindung kann die Reduktion des gegebenenfalls C₅-C₃₄-fettsäurefunktionalisierten, insbesondere gegebenenfalls C₈-C₃₄-fettsäurefunktionalisierten, 3-Oxobuttersäure- Polyglycerinesters (I) mit Wasserstoff als Reduktionsmittel in Gegenwart eines Metalls und/oder einer Metallverbindung auf Basis von Palladium, Platin, Rhodium, Iridium, Ruthenium und Nickel als Katalysator erfolgen.

Gemäß einer alternativen Ausführungsform der vorliegenden Erfindung kann die Reduktion des gegebenenfalls C₅-C₃₄-fettsäurefunktionalisierten, insbesondere gegebenenfalls C₈-C₃₄-fettsäurefunktionalisierten, 3-Oxobuttersäure-Polyglycerinesters (I) mit einem C₁-C₄-Alkohol, insbesondere Isopropanol, als Reduktionsmittel in Gegenwart von Dehydrogenase, insbesondere Alkoholdehydrogenase, als Katalysator erfolgen.

Gemäß einer wiederum alternativen Ausführungsform der vorliegenden Erfindung kann die Reduktion des gegebenenfalls C₅-C₃₄-fettsäurefunktionalisierten, insbesondere gegebenenfalls C₈-C₃₄-fettsäurefunktionalisierten, 3-Oxobuttersäure-Polyglycerinesters (I) autokatalytisch in Gegenwart von Alkali- oder Erdalkaliborhydrid als Reduktionsmittel erfolgen.

Im Rahmen der vorliegenden Erfindung kann die Reduktion der Ketogruppe -C(O) - der Acetylfunktion CH₃ - C(O) - in dem oder den 3-Oxobutyratresten der allgemeinen Formel (I'), wie zuvor definiert, zu einer Hydroxylgruppe mittels chiraler und/oder enantioselektiver Reaktionsführung erfolgen.

In diesem Zusammenhang ist es insbesondere bevorzugt, wenn die Reduktion in Gegenwart eines chiralen und/oder enantioselektiven Katalysators durchgeführt wird. Enantioselektive bzw. chirale Katalysatoren können beispielsweise der zuvor aufgeführte Wilkinson-Katalysator oder eine Dehydrogenase, insbesondere eine Alkoholdehydrogenase, wie das kommerziell erhältliche Chiralidon^{®} R, sein.

Mit anderen Worten kann in diesem Zusammenhang die Reduktion insbesondere enantioselektiv erfolgen.

Insbesondere wird bei dem erfindungsgemäßen Verfahren der gegebenenfalls C₅-C₃₄-fettsäurefunktionalisierte, insbesondere gegebenenfalls C₈-C₃₄-fettsäurefunktionalisierte, 3 3-Hydroxybuttersäure-Polyglycerinester (II) enantiomerenangereichert, insbesondere enantiomerenrein, vorzugsweise in Form des (R)-konfigurierten Enantiomers, gebildet, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃ - C*H(OH) - CH₂- C(O)O -.

In diesem Zusammenhang ist es insbesondere bevorzugt, wenn das (R)-Enantiomer des gegebenenfalls C₅-C₃₄-fettsäurefunktionalisierten, insbesondere gegebenenfalls C₅-C₃₄-fettsäurefunktionalisierten, vorzugsweise gegebenenfalls C₈-C₃₄-fettsäurefunktionalisierten, 3-Hydroxybuttersäure-Polyglycerinesters (II) gebildet wird, bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃ - C*H(OH) - CH₂-C(O)O -.

Die Chiralität beschreibt in der Stereochemie eine räumliche Anordnung von Atomen innerhalb eines Moleküls, bei der einfache Symmetrieoperation, wie beispielsweise eine Spiegelung an einer Symmetrieebene, nicht zu einer Selbstabbildung führt. Bei einem Chiralitätszentrum (synonym auch als Stereozentrum bezeichnet) handelt es sich um einen Punkt (insbesondere um ein Atom) in einem Molekül mit einem Satz an Substituenten in einer solchen räumlichen Anordnung, dass sie mit der spiegelbildlichen Anordnung nicht in Deckung gebracht werden kann. Zueinander spiegelbildlich aufgebauten Moleküle nennt man Enantiomere und ihre jeweiligen chemischen Verbindungen chiral. In der vorliegenden Erfindung liegt das Chiralitätszentrum des 3-Hydroxybutyratrests der allgemeinen Formel (II') am C-Atom in 3-Position, welches eine Hydroxylgruppe als Substituenten aufweist.

Im Rahmen der vorliegenden Erfindung ist unter enantiomerenangereichert das Vorliegen von mindestens 90 %, insbesondere mindestens 95 %, vorzugsweise mindestens 96 %, bevorzugt mindestens 97 %, besonders bevorzugt mindestens 98 %, ganz besonders bevorzugt mindestens 99 %, eines Enantiomers im Reaktionsprodukt zu verstehen. Weiterhin ist im Rahmen der vorliegenden Erfindung unter enantiomerenrein das Vorliegen von im Wesentlichen 100 % eines Enantiomers im Reaktionsprodukt zu verstehen.

Erfindungsgemäß kann aber gemäß einer alternativen Ausführungsform bzw. Vorgehensweise die Reduktion der Ketogruppe - C(O) - der Acetylfunktion CH₃ - C(O) - in dem oder den 3-Oxobutyratresten der allgemeinen Formel (I'), wie zuvor definiert, zu einer Hydroxylgruppe mittels nicht-chiraler und/oder nichtenantioselektiver Reaktionsführung erfolgen.

In diesem Zusammenhang ist es vorteilhaft, wenn die Reduktion in Gegenwart eines nicht-chiralen und/oder nicht-enantioselektiven Katalysators durchgeführt wird. Ein solcher Katalysator ist beispielsweise das zuvor angeführte Raney-Nickel oder Natriumborhydrid (NaBH₄).

Gemäß dieser alternativen Ausführungsform wird insbesondere ein Racemat (racemisches Gemisch) des gegebenenfalls C₅-C₃₄-fettsäurefunktionalisierten, insbesondere gegebenenfalls C₈-C₃₄-fettsäurefunktionalisierten, 3-Hydroxybuttersäure-Polyglycerinesters (II) gebildet, jeweils bezogen auf das mittels Reduktion erzeugte Chiralitätszentrum (asymmetrisches Kohlenstoffatom) des 3-Hydroxybutyratrests der obigen allgemeinen Formel (II'), wie zuvor definiert (d. h. äquimolares Gemisch der beiden möglichen Enantiomere).

Gemäß dieser Ausführungsform wird also ein racemischer gegebenenfalls C₅-C₃₄-fettsäurefunktionalisierter, insbesondere gegebenenfalls C₈-C₃₄-fettsäurefunktionalisierter, (R)/(S)-3-Hydroxybuttersäure-Polyglycerinester (II) gebildet, jeweils bezogen auf das mittels Reduktion erzeugte Chiralitätszentrum (asymmetrisches Kohlenstoffatom) des 3-Hydroxybutyratrests der obigen allgemeinen Formel (II'), wie zuvor definiert.

Erfindungsgemäß wird die Reduktion als eine vollständige Reduktion oder aber als nicht-vollständige Reduktion sämtlicher Ketogruppen - C(O) - der Acetylfunktion CH₃ - C(O) - des mindestens einen 3-Oxobutyratrests der allgemeinen Formel (I') zu einem 3-Hydroxybutyratrest der allgemeinen Formel (II') durchgeführt.

Gemäß einer Ausführungsform der vorliegenden Erfindung wird die Reduktion als eine vollständige Reduktion sämtlicher Ketogruppen - C(O) - der Acetylfunktion CH₃ - C(O) - des mindestens einen 3-Oxobutyratrests der allgemeinen Formel (I'), wie zuvor definiert, zu einem 3-Hydroxybutyratrest der allgemeinen Formel (II'), wie zuvor definiert, durchgeführt werden. Bei dieser Ausführungsform wird also die Reduktion derart durchgeführt, dass sämtliche Ketogruppen - C(O) - der Acetylfunktion CH₃ - C(O) - des mindestens einen 3-Oxobutyratrests der allgemeinen Formel (I'), wie zuvor definiert, zu einem 3-Hydroxybutyratrest der allgemeinen Formel (II'), wie zuvor definiert, umgesetzt werden.

In diesem Zusammenhang wird die Reduktion derart durchgeführt, dass der als Reaktionsprodukt erhältliche gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) keine Acetylfunktion CH₃ - C(O) - mehr aufweist.

In diesem Zusammenhang ist es erfindungsgemäß bevorzugt, wenn mindestens stöchiometrische Mengen an Reduktionsmittel, bezogen auf die zu reduzierenden Ketogruppen des 3-Oxobutyratrests der allgemeinen Formel (I'), eingesetzt werden.

Unter Berücksichtigung von Verfahrensökonomie und Optimierung des Verfahrensablaufs, insbesondere im Hinblick auf die Minimierung von Nebenprodukten, ist es vorteilhaft, wenn das Reduktionsmittel, bezogen auf die Ketogruppen - C(O) - der Acetylfunktion CH₃ - C(O) - des mindestens einen 3-Oxobutyratrests der allgemeinen Formel (I'), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn das Reduktionsmittel einerseits und der zu reduzierende gegebenenfalls C₅-C₃₄-fettsäurefunktionalisierte, insbesondere gegebenenfalls C₈-C₃₄-fettsäurefunktionalisierte, 3-Oxobuttersäure-Polyolester (I) andererseits in einem Molverhältnis von Reduktionsmittel / Ketogruppen - C(O) - der Acetylfunktion CH₃ - C(O) - des 3-Oxobutyratrests der allgemeinen Formel (I'), wie zuvor definiert, in einem Bereich von 1 : 1 bis 10 : 1, insbesondere in einem Bereich von 2 : 1 bis 8 : 1, vorzugsweise in einem Bereich von 3 : 1 bis 6 : 1, eingesetzt werden.

Eine solche vollständige Reduktion ist insbesondere vorteilhaft, da im erhaltenen Reduktionsprodukt keine Restedukte, insbesondere in Form des gegebenenfalls C₅-C₃₄-fettsäurefunktionalisierten, insbesondere gegebenenfalls C₈-C₃₄-fettsäurefunktionalisierten, 3-Oxobuttersäure-Polyglycerinesters (I) mehr vorhanden sind, weshalb eine aufwendige und energieträchtige Aufreinigung zum Erhalt eines reinen Reaktionsprodukts nicht notwendig ist.

Gemäß einer alternativen Ausführungsform der vorliegenden Erfindung wird die Reduktion als nicht-vollständige Reduktion sämtlicher Ketogruppen - C(O) - der Acetylfunktion CH₃ - C(O) - des mindestens einen 3-Oxobutyratrests der allgemeinen Formel (I'), wie zuvor definiert, zu einem 3-Hydroxybutyratrest der allgemeinen Formel (II'), wie zuvor definiert, durchgeführt. Bei dieser Ausführungsform wird also die Reduktion derart durchgeführt werden, dass nicht sämtliche Ketogruppen - C(O) - der Acetylfunktion CH₃ - C(O) - des mindestens einen 3-Oxobutyratrests der allgemeinen Formel (I'), wie zuvor definiert, zu einem 3-Hydroxybutyratrest der allgemeinen Formel (II'), wie zuvor definiert, umgesetzt werden.

Weiterhin ist es in diesem Zusammenhang bevorzugt, wenn die Reduktion derart durchgeführt wird, dass das Reaktionsprodukt mindestens eine Acetylfunktion CH₃ - C(O) - aufweist und/oder dass die Reduktion derart durchgeführt wird, dass das Reaktionsprodukt mindestens einen 3-Oxobutyratrest der allgemeinen Formel (I'), wie zuvor definiert, aufweist.

In diesem Zusammenhang ist es bevorzugt, wenn das Reduktionsmittel, bezogen auf die Ketogruppe - C(O) - der Acetylfunktion CH₃ - C(O) - des mindestens einen 3-Oxobutyratrests der allgemeinen Formel (I'), wie zuvor definiert, in molaren Mengen unterhalb der äquimolaren Menge und/oder in einer unterstöchiometrischen Menge eingesetzt wird.

Mit anderen Worten wird gemäß dieser alternativen Ausführungsform die Reduktion mittels mindestens eines Reduktionsmittels, wie zuvor definiert, unterstöchiometrisch durchgeführt; unterstöchiometrisch in diesem Fall bedeutet, dass ein molarer Überschuss an gegebenenfalls C₅-C₃₄-fettsäurefunktionalisierten, insbesondere gegebenenfalls C₈-C₃₄-fettsäurefunktionalisierten, 3-Oxobuttersäure-Polygylcerinester (I) eingesetzt wird bzw. ein molarer Unterschuss (d. h. sozusagen ein molarer Mangel) an Reduktionsmittel eingesetzt wird, so dass nicht alle Ketogruppen des 3-Oxobutyratrests der allgemeinen Formel (I'), wie zuvor definiert, zu Hydroxylgruppen, wie sie in dem 3-Hydroxybutyratrest der allgemeinen Formel (II') vorliegen, wie zuvor definiert, umgesetzt werden.

Eine solche nicht vollständige Reduktion kann insbesondere vorteilhaft sein, da durch das Vorliegen von sowohl 3-Oxobutyratresten der allgemeinen Formel (I') als auch 3-Hydroxybutyratresten der allgemeinen Formel (II') das Reaktionsprodukt einen Retardierungseffekt aufweist, d. h. durch den unterschiedlich schnellen Abbau der verschiedenen Reste liegt eine unterschiedlich schnelle Verfügbarkeit bzw. Freisetzung des Wirkstoffs 3-Hydroxybuttersäure vor. Insgesamt ist daher eine längerfristige Verfügbarkeit bzw. Freisetzung möglich und somit eine Retard-Therapie mit 3-Hydroxybuttersäure möglich.

Gemäß einer wiederum weiteren besonderen Ausführungsform des erfindungsgemäßen Verfahrens kann das erhaltene Reaktionsprodukt nach erfolgter Reduktion fraktioniert werden, insbesondere destillativ fraktioniert werden.

Auch ist es im Rahmen der vorliegenden Erfindung bevorzugt, wenn nicht umgesetzte Ausgangsverbindungen aus dem Reaktionsprodukt abgetrennt und anschließend rezykliert werden. In diesem Zusammenhang sind die Ausgangsverbindungen der gegebenenfalls C₅-C₃₄-fettsäurefunktionalisierte, insbesondere gegebenenfalls C₈-C₃₄-fettsäurefunktionalisierte, 3-Oxobuttersäure-Polygylcerinester (I) und gegebenenfalls das erfindungsgemäß eingesetzte Reduktionsmittel.

Gemäß des erfindungsgemäßen Verfahrens entspricht der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) der allgemeinen Formel (Ib)

R¹O - CH₂ - CH(OR¹) - CH₂ - [O - CH₂ - CH(OR¹) - CH₂]ₚ - OR¹ (Ib)

wobei in der allgemeinen Formel (Ib)
- die Variable p eine ganze Zahl von 0 bis 6, insbesondere 1 bis 4, vorzugsweise 1 oder 2, besonders bevorzugt 1, darstellt,
- der Rest R¹ jeweils unabhängig voneinander, gleich oder verschieden, darstellt: Wasserstoff, CH₃ - C(O) - CH₂ - C(O) - oder einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, jedoch mit der Maßgabe, dass mindestens ein Rest R¹, insbesondere mindestens zwei Reste R¹, keinen Wasserstoff darstellt, und mit der Maßgabe, dass mindestens ein Rest R¹, insbesondere mindestens zwei Reste R¹, einen Rest CH₃- C(O) - CH₂ - C(O) - darstellt.

Gemäß einer weiteren besonderen Ausführungsform des erfindungsgemäßen Verfahrens kann der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) der allgemeinen Formel (Ic)

R¹O - CH₂ - CH(OR¹) - CH₂ - O - CH₂ - CH(OR¹) - CH₂ - OR¹ (Ic)

entsprechen,
wobei in der allgemeinen Formel (Ic) der Rest R¹ jeweils unabhängig voneinander, gleich oder verschieden, darstellt: Wasserstoff, CH₃ - C(O) - CH₂ - C(O) - oder einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, jedoch mit der Maßgabe, dass mindestens ein Rest R¹, insbesondere mindestens zwei Reste R¹, keinen Wasserstoff darstellt, und mit der Maßgabe, dass mindestens ein Rest R¹, insbesondere mindestens zwei Reste R¹, einen Rest CH₃- C(O) - CH₂ - C(O) - darstellt.

Gemäß einer weiteren besonderen Ausführungsform des erfindungsgemäßen Verfahrens kann der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) der allgemeinen Formel (Id)

R¹O - CH₂ - CH(OR¹) - CH₂ - OR¹ (Id)

entsprechen,
wobei in der allgemeinen Formel (Id) der Rest R¹ jeweils unabhängig voneinander, gleich oder verschieden, darstellt: Wasserstoff, CH₃ - C(O) - CH₂ - C(O) - oder einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, jedoch mit der Maßgabe, dass mindestens ein Rest R¹, insbesondere mindestens zwei Reste R¹, keinen Wasserstoff darstellt, und mit der Maßgabe, dass mindestens ein Rest R¹, insbesondere mindestens zwei Reste R¹, einen Rest CH₃- C(O) - CH₂- C(O) - darstellt.

Insbesondere kann der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) erhältlich sein und/oder erhalten werden durch eine Syntheseroute (A),
wobei gemäß der Syntheseroute (A) in einem ersten Verfahrensschritt mindestens eine Verbindung der allgemeinen Formel (III)

   CH₃- C(O) - CH₂ - C(O)OR³ (III)
wobei in der allgemeinen Formel (III) der Rest R³ ein C₁-C₄-Alkyl, insbesondere Methyl oder Ethyl, bevorzugt Ethyl, darstellt,
mit mindestens einem Polyglycerin umgesetzt wird,
gefolgt von einem zweiten Verfahrensschritt, wobei der zweite Verfahrensschritt
   (i) eine zumindest teilweise Funktionalisierung, insbesondere eine zumindest teilweise Veresterung, noch vorhandener Hydroxylgruppen mittels mindestens einer Carbonsäure und/oder deren Ester oder Anhydrid, vorzugsweise mittels mindestens einer C₅-C₃₄-Fettsäure und/oder deren Ester oder Anhydrid, vorzugsweise mittels mindestens einer C₈-C₃₄-Fettsäure und/oder deren Ester oder Anhydrid, und/oder
   (ii) eine teilweise Umesterung von im ersten Verfahrensschritt eingeführten Estergruppen mittels mindestens einer Carbonsäure und/oder deren Ester oder Anhydrid, vorzugsweise mittels mindestens einer C₅-C₃₄-Fettsäure und/oder deren Ester, vorzugsweise mittels mindestens einer C₈-C₃₄-Fettsäure und/oder deren Ester,
umfasst.

Gleichermaßen kann für den Fall, dass der als Ausgangsverbindung eingesetzte 3-Oxobuttersäure-Polyolester (I), wie zuvor definiert, C₅-C₃₄-fettsäurefunktionalisiert, besonders bevorzugt C₈-C₃₄-fettsäurefunktionalisiert, vorliegt, der als Ausgangsverbindung eingesetzte C₅-C₃₄-fettsäurefunktionalisierte, besonders bevorzugt C₈-C₃₄-fettsäurefunktionalisierte, 3-Oxobuttersäure-Polyglycerinester (I) erhältlich sein und/oder erhalten werden durch eine der beiden nachfolgenden Syntheserouten (A) oder (B),
(A) wobei gemäß einer (ersten) Syntheseroute (A) zunächst in einem ersten Verfahrensschritt mindestens eine Verbindung der allgemeinen Formel (III)

   CH₃-C(O)-CH₂-C(O)OR³ (III)

   wobei in der allgemeinen Formel (III) der Rest R³ ein C₁-C₄-Alkyl, insbesondere Methyl oder Ethyl, bevorzugt Ethyl, darstellt,
   mit mindestens einem Polyglycerin, umgesetzt wird,
   gefolgt von einem zweiten Verfahrensschritt, wobei der zweite Verfahrensschritt
      (i) eine zumindest teilweise Funktionalisierung, insbesondere eine zumindest teilweise Veresterung, noch vorhandener Hydroxylgruppen mittels mindestens einer C₅-C₃₄-Fettsäure und/oder deren Ester oder Anhydrid, vorzugsweise mittels mindestens einer C₈-C₃₄-Fettsäure und/oder deren Ester oder Anhydrid, und/oder
      (ii) eine teilweise Umesterung von im ersten Verfahrensschritt eingeführten Estergruppen mittels mindestens einer C₅-C₃₄-Fettsäure und/oder deren Ester, vorzugsweise mittels mindestens einer C₈-C₃₄-Fettsäure und/oder deren Ester,
   umfasst;
   oder aber
(B) wobei gemäß einer (zweiten, zu (A) alternativen) Syntheseroute (B) zunächst in einem ersten Verfahrensschritt mindestens ein Polyglycerin
   mit mindestens einer C₅-C₃₄-Fettsäure und/oder deren Ester oder Anhydrid, vorzugsweise mit mindestens einer C₈-C₃₄-Fettsäure und/oder deren Ester oder Anhydrid, umgesetzt wird,
   gefolgt von einem zweiten Verfahrensschritt, wobei der zweite Verfahrensschritt
      (i) eine zumindest teilweise Veresterung noch vorhandener Hydroxylgruppen mittels einer Verbindung der allgemeinen Formel (III), wie zuvor definiert, und/oder
      (ii) eine teilweisen Umesterung von im ersten Verfahrensschritt eingeführten Estergruppen mittels einer Verbindung der allgemeinen Formel (III), wie zuvor definiert,
   umfasst.

Weiterhin kann es auch bevorzugt sein, wenn das Polyglycerin der allgemeinen Formel (IVb)

HO - CH₂ - CH(OH) - CH₂ - [O - CH₂ - CH(OH) - CH₂]ₚ - OH (IVb)

entspricht,
wobei in der allgemeinen Formel (IVb) die Variable p eine ganze Zahl von 1 bis 4, vorzugsweise 1 oder 2, besonders bevorzugt 1, darstellt.

Des Weiteren kann es gemäß dem erfindungsgemäßen Verfahren bevorzugt sein, wenn das Polyglycerin ein Diglycerin der Formel (IVc)

HO - CH₂ - CH(OH) - CH₂ - O - CH₂ - CH(OH) - CH₂ - OH (IVc)

ist.

Darüber hinaus kann es gemäß dem erfindungsgemäßen Verfahren vorgesehen sein, wenn das Polyglycerin kein Propan-1 ,2,3-triol (Glycerin) ist.

Im Rahmen des erfindungsgemäßen Verfahrens ist es bevorzugt, wenn die Carbonsäure und/oder deren Ester, vorzugsweise die Fettsäure und/oder der Fettsäureester, eine Carbonsäure und/oder ein Carbonsäureester der allgemeinen Formel (V)

R²-O-R⁴ (V)

ist,
wobei in der allgemeinen Formel (V)
- der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt,
- der Rest R⁴ Wasserstoff oder ein C₁-C₄-Alkyl, insbesondere Wasserstoff, Methyl oder Ethyl, besonders bevorzugt Wasserstoff, darstellt.

Weiterhin ist es im Rahmen des erfindungsgemäßen Verfahrens bevorzugt, wenn das Carbonsäureanhydrid, vorzugsweise das Fettsäureanhydrid ein Carbonsäureanhydrid der allgemeinen Formel (VI)

R²-O-R² (VI)

ist,
wobei in der allgemeinen Formel (VI) der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt.

Im Anschluss an die Reduktion können im Reaktionsprodukt (II) nach erfolgter Reduktion noch vorhandene Hydroxylgruppen zumindest teilweise, vorzugweise vollständig, funktionalisiert, insbesondere verestert, werden.

Gemäß einer besonderen Ausführungsform des erfindungsgemäßen Herstellungsverfahrens kann sich also der Reduktion eine teilweise, insbesondere vollständige Funktionalisierung, insbesondere Veresterung, noch vorhandener Hydroxylgruppen anschließt.

Insbesondere kann es im Rahmen des erfindungsgemäßen Herstellungsverfahrens vorgesehen sein, wenn im Reaktionsprodukt (II) nach erfolgter Reduktion vorhandene Estergruppen, insbesondere in Form von 3-Hydroxybutyratresten gemäß der allgemeinen Formel (II'), wie zuvor definiert, teilweise umgeestert werden.

Erfindungsgemäß kann es insbesondere vorgesehen sein, wenn sich der Reduktion eine teilweise Umesterung vorhandener Estergruppen, insbesondere in Form von 3-Hydroxybutyratresten gemäß der allgemeinen Formel (II'), wie zuvor definiert, anschließt.

In diesem Zusammenhang ist es insbesondere bevorzugt, wenn die Funktionalisierung und/oder Umesterung mittels einer C₅-C₃₄-Fettsäure, bevorzugt einer C₈-C₃₄-Fettsäure, insbesondere in freier Form oder in Form von deren Ester oder Anhydrid, insbesondere wie zuvor definiert, durchgeführt wird.

Gemäß einer besonderen Ausführungsform des erfindungsgemäßen Verfahrens kann die C₅-C₃₄-Fettsäure, bevorzugt die C₈-C₃₄-Fettsäure, insbesondere in freier Form oder in Form von deren Ester oder Anhydrid, ausgewählt sein aus der Gruppe von Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Heneicosansäure, Behensäure, Lignocerinsäure, Cerotinsäure, Montansäure, Melissinsäure, Laccersäure, Geddinsäure, Undecylsäure, Myristoleinsäure, Palmitoleinsäure, Margaroleinsäure, Petroselinsäure, Ölsäure, Elaidinsäure, Vaccensäure, Gadoleinsäure, Cetoleinsäure, Erucasäure, Nervonsäure, Linolsäure, Linolensäuren, Calendulasäure, Punicinsäure, Eleostearinsäuren, Stearidonsäure, Arachidonsäure, Eicosapentaensäure, Docosadiensäure, Docosatetraensäure, Docosapentaensäure, Docosahexaensäure, und Tetracosahexaensäure sowie deren Mischungen.

Gemäß einer weiteren besonderen Ausführungsform des erfindungsgemäßen Verfahrens kann die C₅-C₃₄-Fettsäure, bevorzugt die C₈-C₃₄-Fettsäure, insbesondere in freier Form oder in Form von deren Ester oder Anhydrid, ausgewählt sein aus der Gruppe von Myristinsäure, Pentadecansäure, Palmitoleinsäure, Cetoleinsäure, Ölsäure, Gadoleinsäure, Cetoleinsäure, Erucasäure, Arachidonsäure, Eicosapentaensäure, Docosadiensäure, Docosatetraensäure, Docosapentaensäure, Docosahexaensäure, Tetracosahexaensäure sowie deren Mischungen, vorzugsweise Eicosapentaensäure und Docosahexaensäure sowie deren Mischungen.

Weiterhin kann gemäß einer besonderen Ausführungsformen des erfindungsgemäßen Verfahrens die C₅-C₃₄-Fettsäure, bevorzugt die C₈-C₃₄-Fettsäure, insbesondere in freier Form oder in Form von deren Ester oder Anhydrid, ausgewählt sein aus der Gruppe von fischölbasierten und/oder in Fischölen vorkommenden Fettsäuren, insbesondere Eicosapentaensäure, Docosadiensäure, Docosatetraensäure, Docosapentaensäure, Docosahexaensäure und Tetracosahexaensäure sowie deren Mischungen, vorzugsweise Eicosapentaensäure, Docosahexaensäure sowie deren Mischungen.

Insbesondere wird im Rahmen des erfindungsgemäßen Herstellungsverfahrens während der Reduktion die Position und Anzahl der durch Reduktion erhaltenen Hydroxylgruppen - CH(OH) - des 3-Hydroxybutyratrests der allgemeinen Formel (II'), wie zuvor definiert, im Vergleich zu den zu reduzierenden Ketogruppen - C(O) - des 3-Oxobutyratrests der allgemeinen Formel (I'), wie zuvor definiert, nicht verändert.

Im Rahmen des erfindungsgemäßen Verfahrens ist es bevorzugt, wenn die Reduktion des mindestens einen 3-Oxobutyratrests der allgemeinen Formel (I'), wie zuvor definiert, selektiv nur an der Ketogruppe - C(O) - der Acetylfunktion CH₃ - C(O) - des 3-Oxobutyratrests (I'), wie zuvor definiert, stattfindet. In diesem Zusammenhang ist es bevorzugt, wenn während der Reduktion keine Nebenreaktionen, insbesondere keine Umlagerungen, Abspaltungen, Additionen etc., stattfinden.

Der Begriff "funktionalisiert" bzw. "gegebenenfalls funktionalisiert" bedeutet also, dass freie Hydroxylgruppen (OH-Gruppen), welche sich sowohl am Polyol (IV), als auch am 3-Hydroxybutyratrest der allgemeinen Formel (II') befinden können, gegebenenfalls funktionalisiert sein können.

Erfindungsgemäß ist es vorgesehen, dass die durch die Reduktion gebildeten Hydroxylgruppen (OH-Gruppen) des 3-Hydroxybutyratrests der allgemeinen Formel (II') dieselbe Position im 3-Hydroxybuttersäure-Polyolester (II) vorweisen wie die Ketogruppen - C(O) - der Acetylfunktion CH3 - C(O) - im 3-Oxobutyratrest der allgemeinen Formel (I') vor der Reaktion. In diesem Zusammenhang entspricht die Anzahl der durch Reduktion erzeugten Hydroxylgruppen der Anzahl der reduzierten Ketogruppen.

Insbesondere können bei dem erfindungsgemäßen Herstellungsverfahren als Reaktionsprodukt ein oder mehrere gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) der allgemeinen Formel (IIb)

R⁵O-CH₂-CH(OR⁵)-CH₂-[O-CH₂-CH(OR⁵)-CH₂]ₚ-OR⁵ (IIb)

erhalten werden,
wobei in der allgemeinen Formel (IIb)
   - die Variable p eine ganze Zahl von 1 bis 4, vorzugsweise 1 oder 2, besonders bevorzugt 1, darstellt,
   - der Rest R⁵ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl)-C(O)-, bevorzugt (C₇-C₃₃-Alkyl) - C(O) -, darstellt, oder einen Rest CH₃- CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃-CH(OR⁶)-CH₂ - C(O) - darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer besonderen Ausführungsform des erfindungsgemäßen Verfahrens können als Reaktionsprodukt ein oder mehrere gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) der allgemeinen Formel (Ilc)

R⁵O - CH₂ - CH(OR⁵) - CH₂ - O - CH₂ - CH(OR⁵) - CH₂ - OR⁵ (Ilc)

erhalten werden,
wobei in der allgemeinen Formel (llc) der Rest R⁵ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃- CH(OR⁶) - CH₂ - C(O) - darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer wiederum weiteren Ausführungsform des erfindungsgemäßen Verfahrens kann als Reaktionsprodukt ein Gemisch von mindestens zwei voneinander verschiedenen gegebenenfalls C₅-C₃₄-fettsäurefunktionalisierten, insbesondere gegebenenfalls C₈-C₃₄-fettsäurefunktionalisierten, 3-Hydroxybuttersäure-Polyglycerinestern (II), wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer weiteren besonderen Ausführungsform des erfindungsgemäßen Verfahrens kann als Reaktionsprodukt ein Gemisch von mindestens drei voneinander verschiedenen gegebenenfalls C₅-C₃₄-fettsäurefunktionalisierten, insbesondere gegebenenfalls C₈-C₃₄-fettsäurefunktionalisierten, 3-Hydroxybuttersäure-Polyglycerinestern (II), wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Wie zuvor bereits ausgeführt, kann es erfindungsgemäß vorgesehen sein, wenn im Reaktionsprodukt der 3-Hydroxybutyratrest der allgemeinen Formel (II'), wie zuvor definiert, in Form des (R)-konfigurierten Enantiomers vorliegt.

In diesem Zusammenhang befindet sich das Chiralitätszentrum (asymmetrisches Kohlenstoffatom) an der 3-Position des 3-Hydroxybutyratrests der allgemeinen Formel (II').

Des Weiteren können bei dem erfindungsgemäßen Herstellungsverfahren als Reaktionsprodukt ein oder mehrere 3-Hydroxybuttersäure-Polyglycerinester (II") der allgemeinen Formel (IIb")

R⁷O-CH₂-CH(OR⁷)-CH₂-[O-CH₂-CH(OR⁷)-CH₂]ₚ-OR⁷ (IIb")

erhalten werden,
wobei in der allgemeinen Formel (IIb")
   - die Variable p eine ganze Zahl von 1 bis 4, vorzugsweise 1 oder 2, besonders bevorzugt 1, darstellt,
   - der Rest R⁷ jeweils unabhängig voneinander, gleich oder verschieden, darstellt: Wasserstoff oder CH₃ - CH(OH) - CH₂ - C(O) -, jedoch mit der Maßgabe, dass mindestens ein Rest R¹, insbesondere mindestens zwei Reste R⁷, keinen Wasserstoff darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Darüber hinaus können im Rahmen des erfindungsgemäßen Verfahrens als Reaktionsprodukt ein oder mehrere 3-Hydroxybuttersäure-Polyglycerinester (II") der allgemeinen Formel (llc")

R⁷O-CH₂-CH(OR⁷)-CH₂-O-CH₂-CH(OR⁷)-CH₂-OR⁷ (IIc")

erhalten werden,
wobei in der allgemeinen Formel (llc") der Rest R⁷ jeweils unabhängig voneinander, gleich oder verschieden, Wasserstoff oder CH₃ - CH(OH) - CH₂ - C(O) -, darstellt, jedoch mit der Maßgabe, dass mindestens ein Rest R¹, insbesondere mindestens zwei Reste R⁷, keinen Wasserstoff darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer besonderen Ausführungsform des erfindungsgemäßen Herstellungsverfahrens kann als Reaktionsprodukt ein Gemisch von mindestens zwei voneinander verschiedenen 3-Hydroxybuttersäure-Polyglycerinestern (II"), insbesondere wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer weiteren besonderen Ausführungsform des erfindungsgemäßen Verfahrens kann als Reaktionsprodukt ein Gemisch von mindestens drei voneinander verschiedenen 3-Hydroxybuttersäure-Polyglycerinestern (II"), insbesondere wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Insbesondere kann es im Rahmen der vorliegenden Erfindung vorgesehen sein, wenn im Reaktionsprodukt der 3-Hydroxybutyratrest der allgemeinen Formel (II'), wie zuvor definiert, in Form des (R)-konfigurierten Enantiomers vorliegt.

In diesem Zusammenhang befindet sich das Chiralitätszentrum (asymmetrisches Kohlenstoffatom) in 3-Position des 3-Hydroxybutyratrests der allgemeinen Formel (II').

Insbesondere können bei dem erfindungsgemäßen Herstellungsverfahren als Reaktionsprodukt ein oder mehrere C₅-C₃₄-fettsäurefunktionalisierte, besonders bevorzugt C₈-C₃₄-fettsäurefunktionalisierte, 3-Hydroxybuttersäure-Polyglycerinester (II‴) der allgemeinen Formel (IIb‴)

R⁵O-CH₂-CH(OR⁵)-CH₂-[O-CH₂-CH(OR⁵)-CH₂]ₚ-OR⁵ (IIb‴)

erhalten werden,
wobei in der allgemeinen Formel (IIb‴)
   - die Variable p eine ganze Zahl von 1 bis 4, vorzugsweise 1 oder 2, besonders bevorzugt 1, darstellt,
   - der Rest R⁵ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl)-C(O)-, darstellt, oder einen Rest CH₃- CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃-CH(OR⁶)-CH₂ - C(O) - darstellt, und mit der Maßgabe, dass mindestens ein Rest R⁵ und/oder ein Rest R⁶ einen Rest R² darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Insbesondere kann gemäß einer besonderen Ausführungsform in der vorstehenden allgemeinen Formel (IIb‴) der Rest R⁵ jeweils unabhängig voneinander, gleich oder verschieden, darstellen: einen Rest CH₃- CH(OR⁶) - CH₂ - C(O) -, wobei der Rest R⁶ einen Rest R², wie zuvor definiert, darstellt, oder aber einen Rest R², wie zuvor definiert, jedoch mit der Maßgabe, dass mindestens ein Rest R⁵, insbesondere mindestens zwei Reste R⁵, einen Rest CH₃-CH(OR⁶)-CH₂-C(O)-, wobei der Rest R⁶ einen Rest R², wie zuvor definiert, enthält, darstellt.

Des Weiteren können gemäß einer zusätzlichen besonderen Ausführungsform in der vorstehenden allgemeinen Formel (IIb‴) die Reste R⁵ und R⁶ jeweils unabhängig voneinander, gleich oder verschieden, keinen Wasserstoff darstellen.

Weiterhin können gemäß einer besonderen Ausführungsform des erfindungsgemäßen Verfahren als Reaktionsprodukt ein oder mehrere C₅-C₃₄-fettsäurefunktionalisierte, besonders bevorzugt C₈-C₃₄-fettsäurefunktionalisierte, 3-Hydroxybuttersäure-Polyglycerinester (II‴) der allgemeinen Formel (IIc‴)

R⁵O - CH₂ - CH(OR⁵) - CH₂ - O - CH₂ - CH(OR⁵) - CH₂ - OR⁵ (IIc‴)

erhalten werden,
wobei in der allgemeinen Formel (IIc‴) der Rest R⁵ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃- CH(OR⁶) - CH₂ - C(O) - darstellt, und mit der Maßgabe, dass mindestens ein Rest R⁵ und/oder ein Rest R⁶ einen Rest R² darstellt,
jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer besonderen Ausführungsform kann in der allgemeinen Formel (IIc‴) der Rest R⁵ jeweils unabhängig voneinander, gleich oder verschieden, darstellen: einen Rest CH₃-CH(OR⁶)-CH₂-C(O)-, wobei der Rest R⁶ einen Rest R², wie zuvor definiert, darstellt, oder aber einen Rest R², wie zuvor definiert, jedoch mit der Maßgabe, dass mindestens ein Rest R⁵, insbesondere mindestens zwei Reste R⁵, einen Rest CH₃- CH(OR⁶) - CH₂ - C(O) -, wobei der Rest R⁶ einen Rest R², wie zuvor definiert, enthält, darstellt.

Weiterhin können gemäß einer anderen besonderen Ausführungsform in der allgemeinen Formel (IIc‴) die Reste R⁵ und R⁶ jeweils unabhängig voneinander, gleich oder verschieden, keinen Wasserstoff darstellen.

Insbesondere kann gemäß einer weiteren besonderen Ausführungsform des erfindungsgemäßen Verfahrens als Reaktionsprodukt ein Gemisch von mindestens zwei voneinander verschiedenen C₅-C₃₄-fettsäurefunktionalisierten, besonders bevorzugt C₈-C₃₄-fettsäurefunktionalisierten, 3-Hydroxybuttersäure-Polyglycerinestern (II‴), wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Gemäß einer wiederum weiteren besonderen Ausführungsform des erfindungsgemäßen Verfahrens kann als Reaktionsprodukt ein Gemisch von mindestens drei voneinander verschiedenen C₅-C₃₄-fettsäurefunktionalisierte, besonders bevorzugt C₈-C₃₄-fettsäurefunktionalisierte, 3-Hydroxybuttersäure-Polyglycerinestern (II‴), wie zuvor definiert, erhalten werden, jedoch mit der Maßgabe, dass die Anzahl und Position der durch die Reduktion erhaltenen 3-Hydroxybutyratreste der allgemeinen Formel (II') im 3-Hydroxybuttersäure-Polyolester (II) der Anzahl und Position der zu reduzierenden 3-Oxobutyratreste der allgemeinen Formel (I') im 3-Oxobuttersäure-Polyolester (I) entsprechen.

Wie zuvor bereits ausgeführt kann es gemäß dem erfindungsgemäßen Verfahren vorgesehen sein, wenn im Reaktionsprodukt der 3-Hydroxybutyratrest der allgemeinen Formel (II'), wie zuvor definiert, in Form des (R)-konfigurierten Enantiomers vorliegt.

In diesem Zusammenhang befindet sich das Chiralitätszentrum (asymmetrisches Kohlenstoffatom) in 3-Position des 3-Hydroxybutyratrests der allgemeinen Formel (II').

Wie zuvor bereits ausgeführt, wird üblicherweise das erfindungsgemäße Verfahren in Abwesenheit von Lösemittel und/oder ohne jegliches Lösemittel durchgeführt (d.h. also als Reaktion in Masse bzw. als Reaktion in Substanz bzw. als sogenannte *Bulk Reaction*)*.* Dies hat den Vorteil, dass die erhaltenen Reaktionsprodukte nicht mit Lösemitteln verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten zumindest im Wesentlichen ohne signifikante Nebenproduktbildung.

Eine erfindungsgemäß bevorzugte Vorgehensweise (einschließlich einer möglichen vorgelagerten Synthese der erfindungsgemäß eingesetzten Edukte) ohne nachfolgende Funktionalisierung der erhaltenen Reaktionsprodukte wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden):

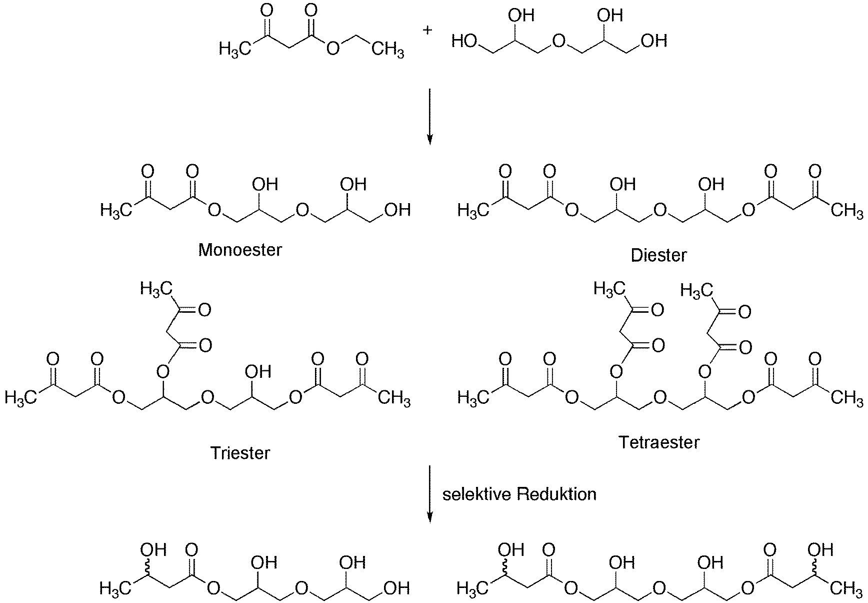

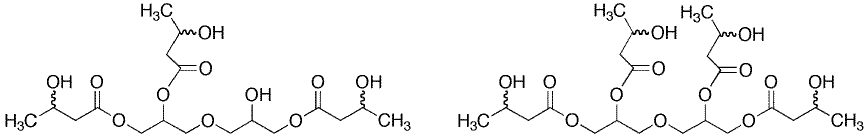

Eine weitere erfindungsgemäß bevorzugte Vorgehensweise (einschließlich einer möglichen vorgelagerten Synthese der erfindungsgemäß eingesetzten Edukte) mit nachfolgender Funktionalisierung der erhaltenen Reaktionsprodukte wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden und wobei in dem nachfolgenden Reaktions- bzw. Syntheseschema der Rest R Wasserstoff oder einen linearen oder verzweigten, gesättigten oder ein- oder mehrfach ungesättigten C₈-C₃₄-Fettsäurerest [= (C₇-C₃₃-Alkyl)-C(O)-Rest] bezeichnet, jedoch mit der Maßgabe, dass pro dargestelltem Molekül mindestens ein Rest R kein H bezeichnet):

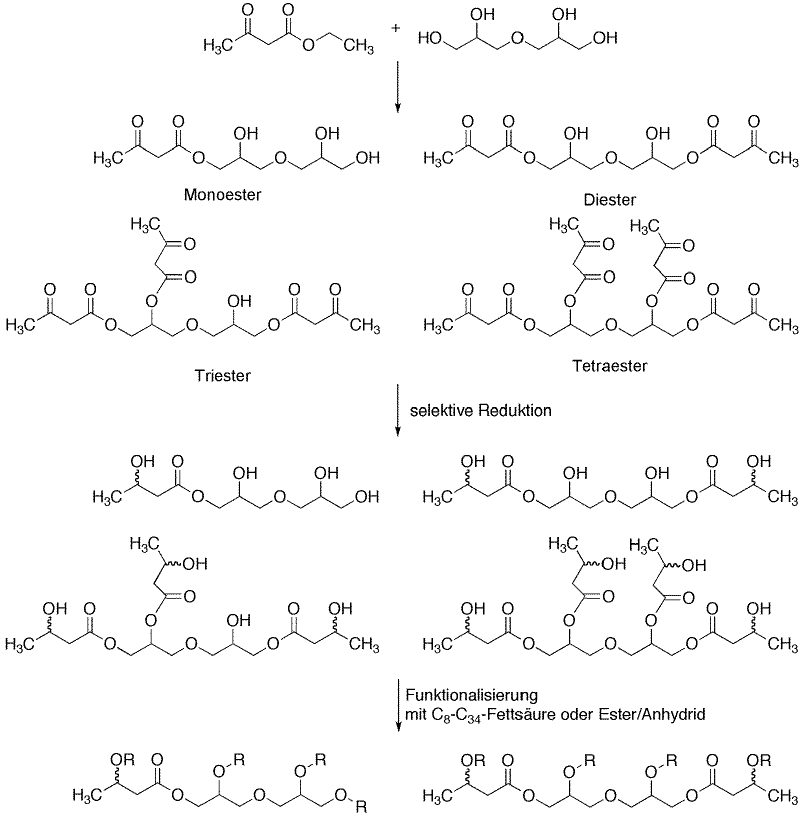

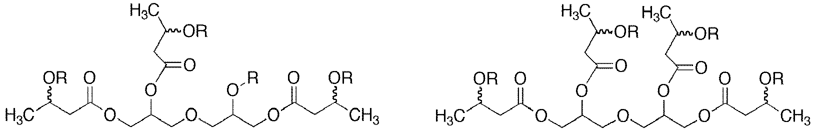

Eine wiederum weitere erfindungsgemäß bevorzugte Vorgehensweise (einschließlich einer möglichen vorgelagerten Synthese der erfindungsgemäß eingesetzten Edukte) unter chiraler Reduktion und mit nachfolgender Funktionalisierung der erhaltenen Reaktionsprodukte ((R)-konfigurierte Enantiomere) wird durch das nachfolgende Reaktions- bzw. Syntheseschema veranschaulicht (wobei in dem nachfolgenden Reaktions- bzw. Syntheseschema der Rest R Wasserstoff oder einen linearen oder verzweigten, gesättigten oder ein- oder mehrfach ungesättigten C₈-C₃₄-Fettsäurerest [= (C₇-C₃₃-Alkyl)-C(O)-Rest] bezeichnet, jedoch mit der Maßgabe, dass pro dargestelltem Molekül mindestens ein Rest R kein H bezeichnet): 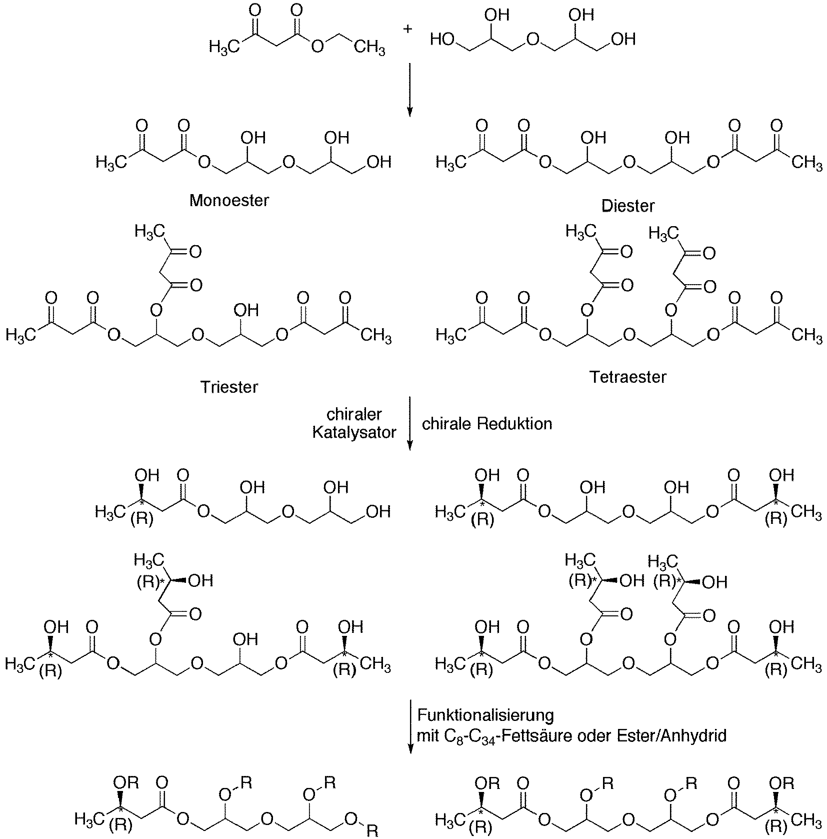 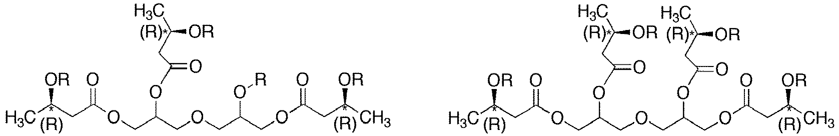 Weiterer Gegenstand - gemäß einem **zweiten** Aspekt der vorliegenden Erfindung - ist das nach dem erfindungsgemäßen Verfahren erhältliche bzw. erfindungsgemäße Reaktionsprodukt (d.h. ein oder mehrere gegebenenfalls C₅-C₃₄-fettsäurefunktionalisierter, insbesondere gegebenenfalls C₈-C₃₄-fettsäurefunktionalisierter, Polyglycerinester von 3-Hydroxybuttersäure oder deren Gemische). Dabei liegt das nach dem erfindungsgemäßen Verfahren erhältliche bzw. erfindungsgemäße Reaktionsprodukt bevorzugt enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH)-CH₂-C(O)O-.

Weiterer Gegenstand der vorliegenden Erfindung ist auch ein gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester (II) der allgemeinen Formel (IIb)

R⁵O-CH₂-CH(OR⁵)-CH₂-[O-CH₂-CH(OR⁵)-CH₂]ₚ-OR⁵ (IIb)

wobei in der allgemeinen Formel (IIb)
- die Variable p eine ganze Zahl von 1 bis 4, vorzugsweise 1 oder 2, besonders bevorzugt 1, darstellt,
- der Rest R⁵ jeweils unabhängig voneinander, gleich oder verschieden, darstellt: Wasserstoff, einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃-CH(OR⁶)-CH₂-C(O)-darstellt.

Gemäß einer besonderen Ausführungsform ist auch Gegenstand der vorliegenden Erfindung ein gegebenenfalls C₅-C₃₄-fettsäurefunktionalisierter 3-Hydroxybuttersäure-Polyglycerinester (II), wie zuvor definiert,
wobei der gegebenenfalls C₅-C₃₄-fettsäurefunktionalisierte 3-Hydroxybuttersäure-Polyglycerinester (II) ein 3-Hydroxybuttersäure-Polyglycerinester der allgemeinen Formel (IIb")

   R⁷O-CH₂-CH(OR⁷)-CH₂-[O-CH₂-CH(OR⁷)-CH₂]ₚ-OR⁷ (IIb")
ist, wobei in der allgemeinen Formel (IIb")
   - die Variable p eine ganze Zahl von 1 bis 4, vorzugsweise 1 oder 2, besonders bevorzugt 1, darstellt,
   - der Rest R⁷ jeweils unabhängig voneinander, gleich oder verschieden, darstellt: Wasserstoff oder CH₃ - CH(OH) - CH₂ - C(O) -, jedoch mit der Maßgabe, dass mindestens ein Rest R¹, insbesondere mindestens zwei Reste R⁷, keinen Wasserstoff darstellt.

Auch ist gemäß einer weiteren besonderen Ausführungsform Gegenstand der vorliegenden Erfindung ein gegebenenfalls C₅-C₃₄-fettsäurefunktionalisierter 3-Hydroxybuttersäure-Polyglycerinester (II), wie zuvor definiert,
wobei der gegebenenfalls C₅-C₃₄-fettsäurefunktionalisierte 3-Hydroxybuttersäure-Polyglycerinester (II) ein C₅-C₃₄-fettsäurefunktionalisierter, besonders bevorzugt C₈-C₃₄-fettsäurefunktionalisierter, 3-Hydroxybuttersäure-Polyglycerinester der allgemeinen Formel (IIb‴)

   R⁵O-CH₂-CH(OR⁵)-CH₂-[O-CH₂-CH(OR⁵)-CH₂]ₚ-OR⁵ (IIb‴)
ist, wobei in der allgemeinen Formel (IIb‴)
   - die Variable p eine ganze Zahl von 1 bis 4, vorzugsweise 1 oder 2, besonders bevorzugt 1, darstellt,
   - der Rest R⁵ jeweils unabhängig voneinander, gleich oder verschieden, darstellt: Wasserstoff, einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃-CH(OR⁶)-CH₂-C(O)-, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃-CH(OR⁶)-CH₂-C(O)-darstellt, und mit der Maßgabe, dass mindestens ein Rest R⁵ und/oder ein Rest R⁶ einen Rest R² darstellt.

Im Rahmen der vorliegenden Erfindung ist unter enantiomerenangereichert das Vorliegen von mindestens 90 %, insbesondere mindestens 95 %, vorzugsweise mindestens 96 %, bevorzugt mindestens 97 %, besonders bevorzugt mindestens 98 %, ganz besonders bevorzugt mindestens 99 %, eines Enantiomers im Reaktionsprodukt zu verstehen. Weiterhin ist im Rahmen der vorliegenden Erfindung unter enantiomerenrein das Vorliegen von im Wesentlichen 100 % eines Enantiomers im Reaktionsprodukt zu verstehen.

Weiterer Gegenstand der vorliegenden Erfindung ist ebenfalls ein gegebenenfalls funktionalisierter 3-Hydroxybuttersäure-Polyolester (II), nämlich ein wie zuvor beschriebener bzw. definierter Polyolester,
wobei der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) der allgemeinen Formel (VIIb)

   R⁸O-CH₂-CH(OR⁸)-CH₂-[O-CH₂-CH(OR⁸)-CH₂]ₚ-OR⁸ (VIIb)

   entspricht,
wobei in der allgemeinen Formel (VIIb)
   - die Variable p eine ganze Zahl von 1 bis 4, vorzugsweise 1 oder 2, besonders bevorzugt 1, darstellt,
   - der Rest R⁸ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- C*H(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁸, insbesondere mindestens zwei Reste R⁸, keinen Wasserstoff darstellt, und mit der Maßgabe, dass mindestens ein Rest R⁸, insbesondere mindestens zwei Reste R⁸, einen Rest CH₃- C*H(OR⁶) - CH₂ - C(O) - darstellt,
wobei der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH)-CH₂-C(O)O-.

Wiederum weiterer Gegenstand der vorliegenden Erfindung ist ebenfalls ein gegebenenfalls funktionalisierter 3-Hydroxybuttersäure-Polyolester (II), nämlich ein wie zuvor beschriebener bzw. definierter Polyolester,
wobei der C₅-C₃₄-fettsäurefunktionalisierte, insbesondere gegebenenfalls C₈-C₃₄-fettsäurefunktionalisierte, 3-Hydroxybuttersäure-Polyglycerinester (II) der allgemeinen Formel (VIIc)

   R⁸O-CH₂-CH(OR⁸)-CH₂-O-CH₂-CH(OR⁸)-CH₂-OR⁸ (VIIc)

   entspricht,
wobei in der allgemeinen Formel (VIIc) der Rest R⁸ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- C*H(OR⁶) - CH₂-C(O)-, wobei der Rest R⁶ Wasserstoff oder einen Rest R², wie zuvor definiert, darstellt, jedoch mit der Maßgabe, dass mindestens ein Rest R⁸, insbesondere mindestens zwei Reste R⁸, keinen Wasserstoff darstellt, und mit der Maßgabe, dass mindestens ein Rest R⁸, insbesondere mindestens zwei Reste R⁸, einen Rest CH₃- C*H(OR⁶) - CH₂ - C(O) - darstellt,
wobei der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH) - CH₂- C(O)O -.

Gemäß einer weiteren besonderen Ausführungsform ist Gegenstand der vorliegenden Erfindung ebenfalls ein 3-Hydroxybuttersäure-Polyolester (II"), nämlich ein wie zuvor beschriebener bzw. definierter Polyolester,
wobei der 3-Hydroxybuttersäure-Polyglycerinester (II") der allgemeinen Formel (VIIb")

   R⁹O-CH₂-CH(OR⁹)-CH₂-[O-CH₂-CH(OR⁹)-CH₂]ₚ-OR⁹ (VIIb")
entspricht,
wobei in der allgemeinen Formel (VIIb")
   - die Variable p eine ganze Zahl von 1 bis 4, vorzugsweise 1 oder 2, besonders bevorzugt 1, darstellt,
   - der Rest R⁹ jeweils unabhängig voneinander, gleich oder verschieden, darstellt: Wasserstoff oder einen Rest CH₃-C*H(OH)-CH₂-C(O)-, jedoch mit der Maßgabe, dass mindestens ein Rest R⁹, insbesondere mindestens zwei Reste R⁹, keinen Wasserstoff darstellt,
wobei der 3 3-Hydroxybuttersäure-Polyglycerinester (II") enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH) - CH₂- C(O)O -.

Wiederum weiterer Gegenstand der vorliegenden Erfindung gemäß einer weiteren Ausführungsform ist ebenfalls ein 3-Hydroxybuttersäure-Polyolester (II"), nämlich ein wie zuvor beschriebener bzw. definierter Polyolester,
wobei der 3-Hydroxybuttersäure-Polyglycerinester der allgemeinen Formel (VIIc")

   R⁹O-CH₂-CH(OR⁹)-CH₂-O-CH₂-CH(OR⁹)-CH₂-OR⁹ (VIIc")

   entspricht,
wobei in der allgemeinen Formel (VIIc") der Rest R⁹ jeweils unabhängig voneinander, gleich oder verschieden, darstellt: Wasserstoff oder einen Rest CH₃ - C*H(OH) - CH₂ - C(O) -, jedoch mit der Maßgabe, dass mindestens ein Rest R⁹, insbesondere mindestens zwei Reste R⁹, keinen Wasserstoff darstellt,
wobei der 3-Hydroxybuttersäure-Polyglycerinester (II") enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH) - CH₂- C(O)O -.

Weiterer Gegenstand gemäß dieser besonderen Ausführungsform der vorliegenden Erfindung ist ebenfalls ein funktionalisierter 3-Hydroxybuttersäure-Polyolester (II‴), nämlich ein wie zuvor beschriebener bzw. definierter Polyolester,
wobei der C₅-C₃₄-fettsäurefunktionalisierte, besonders bevorzugt C₈-C₃₄-fettsäurefunktionalisierte, 3-Hydroxybuttersäure-Polyglycerinester (II‴) der allgemeinen Formel (VIIb‴)

   R⁸O-CH₂-CH(OR⁸)-CH₂-[O-CH₂-CH(OR⁸)-CH₂]ₚ-OR⁸ (VIIb‴)

   entspricht,
wobei in der allgemeinen Formel (VIIb‴)
   - die Variable p eine ganze Zahl von 1 bis 4, vorzugsweise 1 oder 2, besonders bevorzugt 1, darstellt,
   - der Rest R⁸ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- C*H(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁸, insbesondere mindestens zwei Reste R⁸, keinen Wasserstoff darstellt, und mit der Maßgabe, dass mindestens ein Rest R⁸, insbesondere mindestens zwei Reste R⁸, einen Rest CH₃- C*H(OR⁶) - CH₂ - C(O) - darstellt, und mit der Maßgabe, dass mindestens sein Rest R⁸ und/oder ein Rest R⁶ einen Rest R² darstellt,
wobei der C₅-C₃₄-fettsäurefunktionalisierte, besonders bevorzugt C₈-C₃₄-fettsäurefunktionalisierte, 3-Hydroxybuttersäure-Polyglycerinester (II‴) enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH) - CH₂- C(O)O -.

Insbesondere kann gemäß einer besonderen Ausführungsform in der allgemeinen Formel (VIIb‴) der Rest R⁸ jeweils unabhängig voneinander, gleich oder verschieden, darstellen: einen Rest CH₃-C*H(OR⁶)-CH₂-C(O)-, wobei der Rest R⁶ einen Rest R², wie zuvor definiert, darstellt, oder aber einen Rest R², wie zuvor definiert, jedoch mit der Maßgabe, dass mindestens ein Rest R⁸, insbesondere mindestens zwei Reste R⁸, einen Rest CH₃-C*H(OR⁶)-CH₂-C(O) -, wobei der Rest R⁶ einen Rest R², wie zuvor definiert, enthält, darstellt.

Gemäß einer weiteren bevorzugten Ausführungsform können in der allgemeinen Formel (VIIb‴) die Reste R⁸ und R⁶ jeweils unabhängig voneinander, gleich oder verschieden, keinen Wasserstoff darstellen.

Wiederum weiterer Gegenstand der vorliegenden Erfindung gemäß dieser besonderen Ausführungsform ist ebenfalls ein funktionalisierter 3-Hydroxybuttersäure-Polyolester (II‴), nämlich ein wie zuvor beschriebener bzw. definierter Polyolester,
wobei der C₅-C₃₄-fettsäurefunktionalisierte, besonders bevorzugt C₈-C₃₄-fettsäure-funktionalisierte, 3-Hydroxybuttersäure-Polyglycerinester (II‴) der allgemeinen Formel (VIIc‴)

   R⁸O - CH₂ - CH(OR⁸) - CH₂ - O - CH₂ - CH(OR⁸) - CH₂ - OR⁸ (VIIc‴)

   entspricht,
wobei in der allgemeinen Formel (VIIc‴) der Rest R⁸ jeweils unabhängig voneinander, gleich oder verschieden, darstellt: Wasserstoff, einen Rest R², wobei der Rest R² einen Rest vom Typ lineares (geradkettiges) oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃- C*H(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁸, insbesondere mindestens zwei Reste R⁸, keinen Wasserstoff darstellt, und mit der Maßgabe, dass mindestens ein Rest R⁸, insbesondere mindestens zwei Reste R⁸, einen Rest CH₃- C*H(OR⁶) - CH₂ - C(O) - darstellt, und mit der Maßgabe, dass mindestens sein Rest R⁸ und/oder ein Rest R⁶ einen Rest R² darstellt,
wobei der C₅-C₃₄-fettsäurefunktionalisierte, besonders bevorzugt C₈-C₃₄-fettsäurefunktionalisierte, 3-Hydroxybuttersäure-Polyglycerinester (II‴) enantiomerenangereichert, insbesondere enantiomerenrein, vorzugsweise in Form des (R)-konfigurierten Enantiomers, vorliegt, jeweils bezogen auf das mittels Reduktion erzeugte, nachfolgend mit dem Symbol "*" gekennzeichnete Chiralitätszentrum (asymmetrisches Kohlenstoffatom) des 3-Hydroxybutyratrests der allgemeinen Formel (II') CH₃-C*H(OH) - CH₂- C(O)O -.

Gemäß einer besonderen Ausführungsform kann in der allgemeinen Formel (VIIc‴) der Rest R⁸ jeweils unabhängig voneinander, gleich oder verschieden, darstellen: einen Rest CH₃-C*H(OR⁶)-CH₂-C(O)-, wobei der Rest R⁶ einen Rest R², wie zuvor definiert, darstellt, oder aber einen Rest R², wie zuvor definiert, jedoch mit der Maßgabe, dass mindestens ein Rest R⁸, insbesondere mindestens zwei Reste R⁸, einen Rest CH₃- C*H(OR⁶) - CH₂ - C(O) -, wobei der Rest R⁶ einen Rest R², wie zuvor definiert, enthält, darstellt.

Insbesondere können gemäß einer weiteren besonderen Ausführungsform in der allgemeinen Formel (VIIc‴) die Reste R⁸ und R⁶ jeweils unabhängig voneinander, gleich oder verschieden, keinen Wasserstoff darstellen.

Weiterer Gegenstand der vorliegenden Erfindung gemäß diesem Erfindungsaspekt ist entsprechend einer besonderen Ausführungsform ein Gemisch, welches mindestens zwei, vorzugsweise mindestens drei, voneinander verschiedene, gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II), wie zuvor definiert, umfasst.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II), wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieser einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften. Insbesondere die retardierte Freisetzung des physiologisch wirksamen Stoffes im Magen/Darm-Trakt ist im medizinischen Bereich vorteilhaft, da der Wirkstoff 3-Hydroxybuttersäure auf diese Weise über einen längeren Zeitraum zur Verfügung gestellt werden kann und somit eine retardierte Ketokörper-Therapie ermöglicht wird.

Darüber hinaus ist das nach dem erfindungsgemäßen Verfahren erhältliche bzw. erfindungsgemäße Reaktionsprodukt, wie zuvor definiert, und/oder der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II), wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II), wie zuvor definiert, und/oder das nach dem erfindungsgemäßen Herstellungsverfahren erhältliche bzw. erfindungsgemäße Gemisch, wie zuvor definiert, in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II), wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführend erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt, wie zuvor definiert und/oder einen oder mehrere C₅-C₃₄-fettsäurefunktionalisierte, besonders bevorzugt C₈-C₃₄-fettsäurefunktionalisierte, 3-Hydroxybuttersäure-Polyglycerinester, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Auch kann ein nach dem erfindungsgemäßen Herstellungsverfahren erhältliches bzw. erfindungsgemäßes Reaktionsprodukt, wie zuvor definiert und/oder ein oder mehrere C₅-C₃₄-fettsäurefunktionalisierte, besonders bevorzugt C₈-C₃₄-fettsäurefunktionalisierte, 3-Hydroxybuttersäure-Polyolester, wie zuvor definiert, zur prophylaktischen und/oder therapeutischen Behandlung bzw. zur Verwendung bei der prophylaktischen bzw. therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, vorgesehen sein.

Darüber hinaus kann auch die Verwendung eines nach dem erfindungsgemäßen Herstellungsverfahren erhältlichen bzw. erfindungsgemäßen Reaktionsprodukts, wie zuvor definiert, und/oder eines oder mehrerer C₅-C₃₄-fettsäurefunktionalisierter, besonders bevorzugt C₈-C₃₄-fettsäurefunktionalisierter, 3-Hydroxybuttersäure-Polyolester, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, vorgesehen sein.

Des Weiteren kann auch die Verwendung eines nach dem erfindungsgemäßen Herstellungsverfahren erhältlichen bzw. erfindungsgemäßen Reaktionsprodukt, wie zuvor definiert, und/oder eines oder mehrerer C₅-C₃₄-fettsäurefunktionalisierter, besonders bevorzugt C₈-C₃₄-fettsäurefunktionalisierter, 3-Hydroxybuttersäure-Polyolester, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung, vorgesehen sein.

Gleichermaßen weiterer Gegenstand der vorliegenden Erfindung - gemäß einem **vierten** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt, wie zuvor definiert, und/oder ein oder mehrere C₅-C₃₄-fettsäurefunktionalisierte, besonders bevorzugt C₈-C₃₄-fettsäurefunktionalisierte, 3-Hydroxybuttersäure-Polyglycerinester, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich kann auch die Verwendung eines nach dem erfindungsgemäßen Herstellungsverfahren erhältlichen Reaktionsprodukts, wie zuvor definiert, und/oder eines oder mehrerer C₅-C₃₄-fettsäurefunktionalisierter, besonders bevorzugt C₈-C₃₄-fettsäurefunktionalisierter, 3-Hydroxybuttersäure-Polyolester, wie zuvor definiert, in einem Nahrungsmittel- und/oder Lebensmittelerzeugnis vorgesehen sein.

Dabei kann das Nahrungs- und/oder Lebensmittelerzeugnis insbesondere ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nicht-limitierende Durchführungsweise und Ausgestaltung der vorliegenden Erfindung erläutern sollen.

### AUSFÜHRUNGSBEISPIELE:

### Verwendete Abkürzungen

BHB = 3-BHB = 3-Hydroxybuttersäurerest bzw. 3-Hydroxybutyrat-Rest
3-BHB-FS = 3-Hydroxybuttersäure (d. h. freie Säure)
PG(2) = Diglycerin: HO - CH₂ - CH(OH) - CH₂ - O - CH₂- CH(OH) - CH₂ - OH
PG(3) = Polyglycerin: HO - CH₂ - CH(OH) - CH₂ - [O - CH₂ - CH(OH) - CH₂]₂ - OH
DCM = Dichlormethan: CH₂Cl₂
Pd/C = Palladium auf Aktivkohle
Raney-Nickel = feine Körner einer Nickel/Aluminium-Legierung
Wilkinson-Katalysator = Homogenkatalysator mit der Formel Rh(PPh₃)₃Cl

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Beispiel 1

### Herstellung von 3-Hvdroxvbuttersäure-Dialvcerinester-Gemischen aus 3-Oxobuttersäure-Dialvcerinester-Gemischen mittels chiraler Reduktion

In einem 250-ml-Mehrhalskolben werden 100 ml eines Isopropanol/Wasser-Gemischs (9:1) und 5 g eines 3-Oxobuttersäure-Diglycerinester-Gemischs (d. h. Mono-, Di-, Tri- und Tetraglyceride der 3-Oxobuttersäure mit Diglycerin als Polyol) sowie 1 g Chiralidon^{®} R (chiraler Katalysator für die enantioselektive Synthese) vorgelegt.

Das Reaktionsgemisch wird acht Tage bei Raumtemperatur gerührt. Anschließend wird das Enzym abfiltriert und das Reaktionsprodukt getrocknet. Es wird ein entsprechendes Gemisch aus (R)-3-Hydroxybuttersäure-Diglycerinestern (d. h. Mono-, Di-, Tri- und Tetraglyceriden der 3-(R)-Hydroxybuttersäure mit Diglycerin) und geringfügige Mengen an nichtumgesetzten 3-Oxobuttersäure-Diglycerinestern erhalten, welche mit üblichen Methoden abgetrennt werden (chromatographisch oder destillativ). Die Charakterisierung erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Beispiel 2

### Herstellung von 3-Hvdroxvbuttersäure-Dialvcerinester-Gemischen aus 3-Oxobuttersäure-Dialvcerinester-Gemischen mittels nicht-chiraler Reduktion

In einem 300-ml-Druck-Autoklaven werden 50 g eines 3-Oxobuttersäure-Diglycerinester-Gemischs in 100 ml Methanol gelöst und mit 1 bis 5 Gew.% Pd/C versetzt und bei 50 bis 100 °C unter 20 bis 50 bar Wasserstoffdruck für 8 bis 24 h gerührt. Nach Abfiltrieren des Pd/C wird das Methanol abdestilliert. Es wird ein racemisches Gemisch aus (S)/(R)-3-Hydroxybuttersäure-Diglycerinestern und geringen Mengen an nichtumgesetzten 3-Oxobuttersäure-Diglycerinestern erhalten, welche mit üblichen Methoden abgetrennt werden (chromatographisch oder destillativ). Die Charakterisierung erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Beispiel 3

### Herstellung von 3-Hvdroxvbuttersäure-Dialvcerinester-Gemischen aus 3-Oxobuttersäure-Dialvcerinester-Gemischen mittels nicht-chiraler Reduktion

In einem 100-ml-Mehrhalskolben werden 15 g eines 3-Oxobuttersäure-Diglycerinester-Gemischs in 50 ml Ethanol vorgelegt.

Das Reaktionsgemisch wird auf 10 °C gekühlt und es werden schrittweise 1 g Natriumborhydrid (NaBH₄) zugegeben. Nach 30 min wird die Reaktionsmischung durch Zugabe verdünnter Salzsäure neutralisiert. Anschließend wird das Lösemittel entfernt und der Rückstand wird mit wässriger Kaliumhydrogenphosphat-Lösung (K₂HPO₄) aufgenommen und mit Isopropanol extrahiert. Anschließend wird das Lösemittel abdestilliert und der Rückstand in DCM suspendiert. Nach Filtration und erneuter Entfernung des Lösemittels wird ein racemisches Gemisch aus (S)/(R)-3-Hydroxybuttersäure-Diglycerinestern mit geringen Restmengen an 3-Oxobuttersäure-Diglycerin-Monoester erhalten. Der nichtumgesetzte 3-Oxobuttersäure-Diglycerin-Monoester wird unter Vakuum abdestilliert. Die Charakterisierung der Reaktionsprodukte erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Weitere Herstellung von 3-Hvdroxvbuttersäure-Polvolester-Gemischen

Die vorangehenden Versuche werden jeweils wiederholt, jedoch mit anderen 3-Oxobuttersäure-Polyolestern (nämlich mit Glycerin (nicht erfindungsgemäß), Polyglycerin PG(3) und 1,2-Pentandiol (nicht erfindungsgemäß) als Polyolkomponente). Es werden vergleichbare Ergebnisse erhalten. Aufreinigung bzw. Fraktionierung erfolgen in gleicher Weise.

### Funktionalisierungsversuche

Die in den vorangehenden Versuchen erhaltenen Mono-/Di-/Tri-/Tetra-Diglycerinester-Gemische sowie die chromatographisch hieraus abgetrennten einzelnen Diglycerinester in Reinform werden jeweils nachfolgend durch Umsetzung mit Docosahexaensäure-Anhydrid funktionalisiert, um an noch verbliebenen (d. h. an noch freien) OH-Gruppen des 3-BHB-Rests und/oder des Polyolrests jeweils eine Veresterung mit Docosahexaensäure durchzuführen. Das diesbezügliche allgemeine Reaktionsschema hierzu ist im allgemeinen Beschreibungsteil dargestellt und erläutert.

Die Versuche zeigen, dass die beabsichtigte Funktionalisierung durch Umsetzung mit Docosahexaensäure-Anhydrid zu den gewünschten funktionalisierten (d. h. fettsäurefunktionalisierten bzw. fettsäureveresterten) Produkten führt (d. h. Veresterung von freien OH-Gruppen des 3-BHB-Rests und/oder des Polyolrests mit Docosahexaensäure), wie durch entsprechende Analytik bestätigt.

Vergleichbare Funktionalisierungsversuche werden auch mit Eicosapentaensäure-Anhydrid einerseits und mit einem Gemisch von Docosahexaensäure-Anhydrid/ Eicosapentaensäure-Anhydrid andererseits durchgeführt und führen zu analogen Ergebnissen (d. h. jeweils Veresterung von freien OH-Gruppen des 3-BHB-Rests und/oder des Polyolrests), wie durch entsprechende Analytik bestätigt. Die Versuche zeigen, dass die beabsichtigte Funktionalisierung auch durch Umsetzung mit Eicosapentaensäure-Anhydrid sowie mit dem Gemisch von Docosahexaensäure-Anhydrid/ Eicosapentaensäure-Anhydrid zu den gewünschten funktionalisierten (d. h. fettsäurefunktionalisierten bzw. fettsäureveresterten) Produkten führt (d. h. Veresterung von freien OH-Gruppen des 3-BHB-Rests und/oder des Polyolrests), wie durch entsprechende Analytik bestätigt.

### Weitere Funktionalisierungsversuche

Die in den vorangehenden Versuchen erhaltenen Mono-/Di-/Tri-/Tetra-Diglycerinester-Gemische sowie die chromatographisch hieraus abgetrennten einzelnen Diglycerinester in Reinform werden jeweils nachfolgend durch Umsetzung mit Docosahexaensäure funktionalisiert, um die an freien OH-Gruppen des 3-BHB-Rests und/oder des Polyolrests veresterten, fettsäurefunktionalisierten bzw. fettsäureveresterten Produkte zu erhalten.

Die Versuche zeigen, dass die beabsichtigte Funktionalisierung durch Umsetzung mit Docosahexaensäure zu den gewünschten Produkten führt (d. h. Veresterung von freien OH-Gruppen des 3-BHB-Rests und/oder des Polyolrests), wie durch entsprechende Analytik bestätigt.

Vergleichbare Funktionalisierungsversuche werden jeweils auch mit Eicosapentaensäure und Ölsäure durchgeführt und führen zu analogen Ergebnissen (d. h. Veresterung von freien OH-Gruppen des 3-BHB-Rests und/oder des Polyolrests), wie durch entsprechende Analytik bestätigt. Die Versuche zeigen, dass die beabsichtigte Funktionalisierung auch durch Umsetzung jeweils mit Eicosapentaensäure und Ölsäure zu den gewünschten Produkten führt (d. h. Veresterung der freien OH-Gruppen), wie durch entsprechende Analytik bestätigt.

### Weitere Herstellung von nicht-funktionalisierten und funktionalisierten 3-BHB-Polyolester-Gemischen

a) Alle vorangehenden Versuche werden jeweils wiederholt, jedoch mit anderen 3-Oxobuttersäure-Polyolestern (nämlich jeweils mit 3-Oxobuttersäure-Polyglycerinester PG(3) und mit 3-Oxobuttersäure-1,2-Pentandiolester (nicht erfindungsgemäß)). Die vorgenannten Polyolester 3-Oxobuttersäure-Polyglycerinester PG(3) und 3-Oxobuttersäure-1,2-Pentandiolester werden sowohl katalytisch (chiral und nicht-chiral), als auch autokatalytisch effizient zu den gewünschten Produkten (d. h. den entsprechenden 3-Hydroxybuttersäure-Polyolestern) umgesetzt. Es werden vergleichbare Ergebnisse zu den vorangehenden Versuchen erhalten. Aufreinigung und Trennung bzw. Fraktionierung und Analytik erfolgen in gleicher Weise. Anschließend werden Funktionalisierungen in der zuvor beschriebenen Weise durchgeführt, welche zu den entsprechenden Ergebnissen führen.
b) Die Versuche mit 3-Oxobuttersäure-Polyolestern jeweils mit 1,2-Pentandiol (nicht erfindungsgemäß) oder Diglycerin oder Polyglycerin PG(3) als Polyol werden mit Raney-Nickel als Katalysator und Wasserstoff als Reduktionsmittel bei Temperaturen zwischen 75 und 110 °C wiederholt. Es werden vergleichbare Ergebnisse erhalten. Aufreinigung, Trennung bzw. Fraktionierung und Analytik erfolgen in gleicher Weise. Anschließend werden Funktionalisierungen in der zuvor beschriebenen Weise durchgeführt, welche zu den entsprechenden Ergebnissen führen.
c) Die Versuche mit 3-Oxobuttersäure-Polyolestern mit 1,2-Pentandiol (nicht erfindungsgemäß), Diglycerin und Polyglycerin PG(3) als Polyol werden mit dem Wilkinson-Katalysator (Rh(PPh₃)₃Cl) als Katalysator und Wasserstoff als Reduktionsmittel bei Temperaturen zwischen 75 und 110 °C wiederholt. Es werden vergleichbare Ergebnisse erhalten. Aufreinigung, Trennung bzw. Fraktionierung und Analytik erfolgen in gleicher Weise. Anschließend werden Funktionalisierungen in der zuvor beschriebenen Weise durchgeführt, welche zu den entsprechenden Ergebnissen führen.
d) Da insbesondere die 3-BHB-Diglycerinester ein nur wenig bitteren Geschmack aufweisen, sind vor allem diese Ester eine effiziente Produktgruppe für eine therapeutische Anwendung. Daher wird die Reduktion eines 3-Oxobuttersäure-Diglycerinester-Gemischs in einem größeren Maßstab (2 bis 4 kg) durchgeführt.
   Zunächst werden in einem Maßstab von 2 kg die stöchiometrischen Reaktionsbedingungen des vorangehenden Versuchs mit Chiralidon^{®} R als Katalysator und Isopropanol als Reduktionsmittel angewendet. Nach 10 Tagen Rühren bei Raumtemperatur wird ein vollständig reduziertes Reaktionsprodukt (d. h. 3-Hydroxybuttersäure-Diglycerinester-Gemisch) erhalten. Anschließend folgt die Abtrennung des Enzyms und eine destillative Reinigung.
   Die Hälfte dieses Reaktionsprodukts (ca. 1 kg) wird mit Docosahexaensäure-Anhydrid (200 Mol-% Überschuss) vollständig funktionalisiert. Analysen bestätigen, dass im funktionalisierten Produkt keine freie Hydroxylgruppen mehr vorliegen. Nach dem Abdestillieren des überschüssigen Fettsäureanhydrids wird ein betreffendes vollständig funktionalisiertes Gemisch von 3-Hydroxybuttersäure-Diglycerinestern erhalten.
   Das vollständig funktionalisierte Gemisch von Diglycerinestern der 3-Hydroxybuttersäure weist nur einen leicht bitteren Geschmack auf und ist organoleptisch akzeptabel und kompatibel.

### Herstellung von 3-Oxobuttersäure-Diglycerinestern (= Edukt)

In einem 100-ml-Mehrhalskolben mit Dephlegmator (Partialkondensator) und Destillationsbrücke werden 25 g 3-Oxobuttersäureethylester (Ethylacetoacetat bzw. Acetessigester) und 6,5 g Diglycerin vorgelegt.

Bei einer Temperatur von 50 °C werden 0,35 g immobilisiertes Enzym (CALB-Lipase auf Polymerträger, abgeleitet von *Candida antarctica,* z. B. Novozym^{®} 435) zugegeben. Das Reaktionsgemisch wird bei 50 °C für 24 h gerührt. Anschließend wir das Enzym abfiltriert und der überschüssige 3-Oxobuttersäureethylester unter Vakuum abdestilliert.

Das erhaltene Reaktionsprodukt ist ein 3-Oxobuttersäure-Diglycerin-Ester und besteht nach analytischer Untersuchung aus folgender Zusammensetzung: 3-Oxobuttersäure-Mono-Diglycerinester 45 %, 3-Oxobuttersäure-Di-Diglycerinester 48 %, 3-Oxobuttersäure-Tri-Diglycerinester 7 %. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner 3-Oxobuttersäure-Mono-Diglycerinester, reiner 3-Oxobuttersäure-Di-Diglycerinester und reiner 3-Oxobuttersäure-Tri-Diglycerinester). Ein anderer Teil des Gemischs wird einer Auftrennung mittels fraktionierter Destillation unterzogen.

### Weitere Herstellung von 3-Oxobuttersäure-Diglycerinestern (= Edukt)

In einem 250-ml-Mehrhalskolben mit Dephlegmator (Partialkondensator) und Destillationsbrücke werden 106 g 3-Oxobuttersäureethylester (Ethylacetoacetat bzw. Acetessigester) und 29 g Diglycerin vorgelegt.

Bei einer Temperatur von 100 °C werden 1,4 g 30 %-ige methanolische NaOMe-Lösung unter Rühren zugegeben. Das bei der Reaktion gebildete Ethanol wird kontinuierlich abdestilliert. Nach einer Reaktionszeit von 5 h wird das Reaktionsgemisch abgekühlt und mit NaCl-Lösung gewaschen. Die Rohestermischung wird anschließend getrocknet und der überschüssige 3-Oxobuttersäureethylester unter Vakuum abdestilliert.

Das Reaktionsprodukt ist ein 3-Oxobuttersäure-Diglycerin-Ester mit folgender Zusammensetzung: 3-Oxobuttersäure-Mono-Diglycerinester 26 %, 3-Oxobuttersäure-Di-Diglycerinester 51 %, 3-Oxobuttersäure-Tri-Diglycerinester 22 %, 3-Oxobuttersäure-Tetra-Diglycerinester 1 %. Die Charakterisierung erfolgt mittels GC, GPC und GC-MS.

Im Rahmen der Aufreinigung werden Edukte und Reaktionsnebenprodukte entfernt, so dass ein Reingemisch erhalten wird. Ein Teil des Gemischs wird einer Auftrennung mittels Chromatographie unterzogen, so dass die verschiedenen Diglycerinester jeweils als Reinstoffe erhalten werden (d. h. jeweils reiner 3-Oxobuttersäure-Mono-Diglycerinester, reiner 3-Oxobuttersäure-Di-Diglycerinester, reiner 3-Oxobuttersäure-Tri-Diglycerinester etc.). Ein anderer Teil des Gemischs wird einer Auftrennung mittels fraktionierter Destillation unterzogen.

### Physiologische Anwendungsversuche: in-vitro-Verdauversuche

### Verdauversuche (Spalt- bzw. Sgaltungsversuche) von erfindungsgemäßen 3-BHB-Diglycerinester-Gemischen

Mittels Spaltungsversuchen wird gezeigt, dass erfindungsgemäß hergestellte 3-BHB-Diglycerinester bzw. deren Gemische sowie deren funktionalisierte Derivate/Analog im menschlichen gastrointestinalen Trakt gespalten werden können.

Als Ausgangsgemisch wird einerseits ein aufgereinigtes, nach dem erfindungsgemäßen Verfahren erhaltenes enantiomerenreines Gemisch aus jeweils (R)-konfiguriertem 3-Hydroxybuttersäure-Mono-Diglycerinester, 3-Hydroxybuttersäure-Di-Diglycerinester, 3-Hydroxybuttersäure-Tri-Diglycerinester und 3-Hydroxybuttersäure-Tetra-Diglycerinester und andererseits ein aufgereinigtes, nach dem erfindungsgemäßen Verfahren erhaltenes, enantiomerenreines fettsäurefunktionalisiertes Gemisch aus jeweils (R)-konfiguriertem 3-Hydroxybuttersäure-Mono-Diglycerinester, 3-Hydroxybuttersäure-Di-Diglycerinester, 3-Hydroxybuttersäure-Tri-Diglycerinester und 3-Hydroxybuttersäure-Tetra-Diglycerinester eingesetzt.

Für die Spaltungsversuche unter köpernahen Bedingungen werden jeweils zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei allen Experimenten ist zu erkennen, dass sich die Kaskade (Tetraester wird zu Triester, Triester wird zu Diester etc.) bis zu der gewünschten freien Säure 3-Hydroxybuttersäure (3-BHB-FS) fortsetzt.

### Weitere Verdauversuche (Spaltungsversuche) von erfindungsgemäßen 3-BHB-Diglycerinester-Gemischen

### Spaltungsversuche mit Pankreatin

2 g eines wie zuvor beschrieben hergestellten enantiomerenreinen Gemischs auf Basis von jeweils (R)-konfiguriertem 3-Hydroxybuttersäure-Mono-Diglycerinester, 3-Hydroxybuttersäure-Di-Diglycerinester, 3-Hydroxybuttersäure-Tri-Diglycerinester und 3-Hydroxybuttersäure-Tetra-Diglycerinester und ein entsprechend fettsäurefunktionalisiertes Gemisch werden jeweils in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt jeweils über den Beobachtungszeitraum an (Spaltung des fettsäurefunktionalisierten 3-Hydroxybuttersäure-Diglycerinester-Gemischs zu der freien Säure). Der Umsatz/ Zeit-Verlauf der wässrigen Spaltung des erfindungsgemäßen Estergemischs mittels Pankreatin, einschließlich Zunahme der Säurezahl über die Zeit, belegt die gewünschte Zersetzung des Eduktgemischs zu der freien Säure. Dies wird durch entsprechende Analytik bestätigt. Der Versuch belegt, dass das erfindungsgemäße Ausgangsgemisch ein geeigneter physiologischer Präkursor für 3-Hydroxybuttersäure für die entsprechenden Ketokörpertherapien darstellt. Der Versuch wird jeweils anhand der einzelnen Ester in Reinform wiederholt und verifiziert. Es werden vergleichbare Ergebnisse erhalten, d. h. sowohl der 3-Hydroxybuttersäure-Mono-Diglycerinester als auch der 3-Hydroxybuttersäure-Di-Diglycerinester sowie auch der 3-Hydroxybuttersäure-Tri-Diglycerinester und der 3-Hydroxybuttersäure-Tetra-Diglycerinester als auch deren funktionalisierte Derivate werden durch Pankreatin jeweils zu der freien 3-Hydroxybuttersäure (3-BHB-FS) gespalten.

Die zuvor geschilderten Spaltungsversuche belegen, dass die bevorzugt enantiomerenreinen und gegebenenfalls funktionalisierten, vorzugsweise gegebenenfalls fettsäurefunktionalisierten, bevorzugt gegebenenfalls C₅-C₃₄-fettsäurefunktionalisierten, insbesondere gegebenenfalls C₈-C₃₄-fettsäurefunktionalisierten, Polyolester, insbesondere Polyglycerinester, der 3-Hydroxybuttersäure effiziente Präkursoren bzw. Metabolite der freien 3-Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

## Patentansprüche

1. Verfahren zur Herstellung eines gegebenenfalls C₅-C₃₄-fettsäurefunktionalisierten, insbesondere gegebenenfalls C₈-C₃₄-fettsäurefunktionalisierten, Polyglycerinesters von 3-Hydroxybuttersäure,
wobei ein gegebenenfalls C₅-C₃₄-fettsäurefunktionalisierter, insbesondere gegebenenfalls C₈-C₃₄-fettsäurefunktionalisierter, 3-Oxobuttersäure-Polyglycerinester (I), welcher mindestens einen 3-Oxobutyratrest (3-Oxobuttersäurerest) der allgemeinen Formel (I')
CH₃ - C(O) - CH₂ - C(O)O - (I')
aufweist, mittels mindestens eines Reduktionsmittels einer selektiven Reduktion des mindestens einen 3-Oxobutyratrests der allgemeinen Formel (I') an der Ketogruppe - C(O) - der Acetylfunktion CH₃ - C(O) - zu einem 3-Hydroxybutyratrest (3-Hydroxybuttersäurerest) der allgemeinen Formel (II')
CH₃ - CH(OH) - CH₂ - C(O)O - (II')
unterzogen wird,
wobei der als Ausgangsverbindung eingesetzte, gegebenenfalls C₅-C₃₄-fettsäurefunktionalisierte, insbesondere gegebenenfalls C₈-C₃₄-fettsäurefunktionalisierte, 3-Oxobuttersäure-Polyglycerinester (I) der allgemeinen Formel (Ib)
R¹O - CH₂ - CH(OR¹) - CH₂ - [O - CH₂ - CH(OR¹) - CH₂]ₚ- OR¹ (Ib)
entspricht,
wobei in der allgemeinen Formel (Ib)
• die Variable p eine ganze Zahl von 1 bis 4, vorzugsweise 1 oder 2, besonders bevorzugt 1, darstellt,
• der Rest R¹ jeweils unabhängig voneinander, gleich oder verschieden, darstellt: Wasserstoff, CH₃ - C(O) - CH₂ - C(O) - oder einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl)- C(O) -, darstellt, jedoch mit der Maßgabe, dass mindestens ein Rest R¹, insbesondere mindestens zwei Reste R¹, keinen Wasserstoff darstellt, und mit der Maßgabe, dass mindestens ein Rest R¹, insbesondere mindestens zwei Reste R¹, einen Rest CH₃- C(O) - CH₂ - C(O) - darstellt; und
wobei das Reduktionsmittel ausgewählt ist aus der Gruppe von Wasserstoff, Hydriden und Alkoholen sowie deren Mischungen; und
wobei die Reduktion als eine vollständige Reduktion oder aber als nicht-vollständige Reduktion sämtlicher Ketogruppen - C(O) - der Acetylfunktion CH₃ - C(O) - des mindestens einen 3-Oxobutyratrests der allgemeinen Formel (I') zu einem 3-Hydroxybutyratrest der allgemeinen Formel (II') durchgeführt wird;
so dass als Reaktionsprodukt ein gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester (II) erhalten wird.

2. Verfahren nach Anspruch 1,
wobei das Reduktionsmittel ausgewählt ist aus der Gruppe von Wasserstoff, Hydriden, insbesondere anorganischen Hydriden, und Alkoholen, insbesondere C₁-C₄-Alkoholen, sowie deren Mischungen, vorzugsweise ausgewählt aus der Gruppe von Wasserstoff, Alkali- oder Erdalkaliborhydrid, Alkali- oder Erdalkalihydrid, Alkali- oder Erdalkalialuminiumhydrid, Methanol, Ethanol, Propanol und Isopropanol sowie deren Mischungen, bevorzugt aus der Gruppe von Wasserstoff, Alkali- oder Erdalkaliborhydrid und Isopropanol.

3. Verfahren nach Anspruch 1 oder Anspruch 2,
wobei die Reduktion autokatalytisch oder in Gegenwart eines Katalysators durchgeführt wird;
insbesondere wobei im Fall der Durchführung der Reduktion in Gegenwart eines Katalysators der Katalysator nach erfolgter Reduktion rezykliert wird.

4. Verfahren nach einem der Ansprüche 1 bis 3,
wobei die Reduktion in Gegenwart eines Katalysators durchgeführt wird; insbesondere wobei der Katalysator nach erfolgter Reduktion rezykliert wird; und/oder insbesondere wobei der Katalysator ausgewählt ist aus Enzymen und/oder Metallen oder Metallverbindungen, insbesondere Edelmetallen und Übergangsmetallverbindungen; und/oder
insbesondere wobei der Katalysator ausgewählt ist aus der Gruppe von Dehydrogenasen, insbesondere Alkoholdehydrogenasen, und Metallen und/oder Metallverbindungen auf Basis von Palladium, Platin, Rhodium, Iridium, Ruthenium und Nickel, vorzugsweise aus der Gruppe von Dehydrogenasen, insbesondere Alkoholdehydrogenasen, und Metallen und/oder Metallverbindungen auf Basis von Platin, Palladium, Nickel und Rhodium.

5. Verfahren nach einem der Ansprüche 1 bis 4,
wobei die Reduktion derart durchgeführt wird, dass der als Reaktionsprodukt erhältliche gegebenenfalls C₅-C₃₄-fettsäurefunktionalisierte, insbesondere gegebenenfalls C₈-C₃₄-fettsäurefunktionalisierte, 3-Hydroxybuttersäure-Polyolester (II), insbesondere 3-Hydroxybuttersäure-Polyglycerinester, keine Acetylfunktion CH₃ - C(O) -aufweist; und/oder
wobei das Reduktionsmittel, bezogen auf die Ketogruppen - C(O) - der Acetylfunktion CH₃- C(O) - des mindestens einen 3-Oxobutyratrests der allgemeinen Formel (I'), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei das Reduktionsmittel einerseits und der zu reduzierende gegebenenfalls C₅-C₃₄-fettsäurefunktionalisierte, insbesondere gegebenenfalls C₈-C₃₄-fettsäurefunktionalisierte, 3-Oxobuttersäure-Polyolester (I) andererseits in einem Molverhältnis von Reduktionsmittel / Ketogruppen - C(O) - der Acetylfunktion CH₃ - C(O) - des 3-Oxobutyratrests der allgemeinen Formel (I'), wie zuvor definiert, in einem Bereich von 1 : 1 bis 10 : 1, insbesondere in einem Bereich von 2 : 1 bis 8 : 1, vorzugsweise in einem Bereich von 3 : 1 bis 6 : 1, eingesetzt werden.

6. Verfahren nach einem der Ansprüche 1 bis 4,
wobei die Reduktion derart durchgeführt wird, dass das Reaktionsprodukt mindestens eine Acetylfunktion CH₃ - C(O) - aufweist und/oder wobei die Reduktion derart durchgeführt wird, dass das Reaktionsprodukt mindestens einen 3-Oxobutyratrest der allgemeinen Formel (I') aufweist; und/oder
wobei das Reduktionsmittel, bezogen auf die Ketogruppen - C(O) - der Acetylfunktion CH₃ - C(O) - des mindestens einen 3-Oxobutyratrests der allgemeinen Formel (I'), in molaren Mengen unterhalb der äquimolaren Menge und/oder in einer unterstöchiometrischen Menge eingesetzt wird.

7. Verfahren nach einem der vorangehenden Ansprüche,
wobei im Reaktionsprodukt (II) nach erfolgter Reduktion noch vorhandene Hydroxylgruppen zumindest teilweise, vorzugsweise vollständig, funktionalisiert, insbesondere verestert, werden; und/oder
wobei sich der Reduktion eine teilweise, insbesondere vollständige Funktionalisierung, insbesondere Veresterung, noch vorhandener Hydroxylgruppen anschließt; und/oder
wobei im Reaktionsprodukt (II) nach erfolgter Reduktion vorhandene Estergruppen, insbesondere in Form von 3-Hydroxybutyratresten gemäß der allgemeinen Formel (II'), wie zuvor definiert, teilweise umgeestert werden;
wobei sich der Reduktion eine teilweise Umesterung vorhandener Estergruppen, insbesondere in Form von 3-Hydroxybutyratresten gemäß der allgemeinen Formel (II'), wie zuvor definiert, anschließt;
insbesondere wobei die Funktionalisierung und/oder Umesterung mittels einer C₅-C₃₄-Fettsäure, bevorzugt einer C₈-C₃₄-Fettsäure, insbesondere in freier Form oder in Form von deren Ester oder Anhydrid, durchgeführt wird; und/oder
insbesondere wobei die C₅-C₃₄-Fettsäure, bevorzugt die C₈-C₃₄-Fettsäure, insbesondere in freier Form oder in Form von deren Ester oder Anhydrid, ausgewählt ist aus der Gruppe von Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Heneicosansäure, Behensäure, Lignocerinsäure, Cerotinsäure, Montansäure, Melissinsäure, Laccersäure, Geddinsäure, Undecylsäure, Myristoleinsäure, Palmitoleinsäure, Margaroleinsäure, Petroselinsäure, Ölsäure, Elaidinsäure, Vaccensäure, Gadoleinsäure, Cetoleinsäure, Erucasäure, Nervonsäure, Linolsäure, Linolensäuren, Calendulasäure, Punicinsäure, Eleostearinsäuren, Stearidonsäure, Arachidonsäure, Eicosapentaensäure, Docosadiensäure, Docosatetraensäure, Docosapentaensäure, Docosahexaensäure, und Tetracosahexaensäure sowie deren Mischungen; und/oder
insbesondere wobei die C₅-C₃₄-Fettsäure, bevorzugt die C₈-C₃₄-Fettsäure, insbesondere in freier Form oder in Form von deren Ester oder Anhydrid, ausgewählt ist aus der Gruppe von Myristinsäure, Pentadecansäure, Palmitoleinsäure, Cetoleinsäure, Ölsäure, Gadoleinsäure, Cetoleinsäure, Erucasäure, Arachidonsäure, Eicosapentaensäure, Docosadiensäure, Docosatetraensäure, Docosapentaensäure, Docosahexaensäure, Tetracosahexaensäure sowie deren Mischungen, vorzugsweise Eicosapentaensäure und Docosahexaensäure sowie deren Mischungen; und/oder
insbesondere wobei die C₅-C₃₄-Fettsäure, bevorzugt die C₈-C₃₄-Fettsäure, insbesondere in freier Form oder in Form von deren Ester oder Anhydrid, ausgewählt ist aus der Gruppe von fischölbasierten und/oder in Fischölen vorkommenden Fettsäuren, insbesondere Eicosapentaensäure, Docosadiensäure, Docosatetraensäure, Docosapentaensäure, Docosahexaensäure und Tetracosahexaensäure sowie deren Mischungen, vorzugsweise Eicosapentaensäure, Docosahexaensäure sowie deren Mischungen.

8. Reaktionsprodukt, nämlich gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester (II), erhältlich gemäß dem Verfahren nach einem der vorangehenden Ansprüche.

9. Gegebenenfalls C₅-C₃₄-fettsäurefunktionalisierter, insbesondere gegebenenfalls C₈-C₃₄-fettsäurefunktionalisierter, 3-Hydroxybuttersäure-Polyglycerinester (II) der allgemeinen Formel (IIb)
R⁵O - CH₂ - CH(OR⁵) - CH₂ - [O - CH₂ - CH(OR⁵) - CH₂]ₚ - OR⁵ (IIb)
wobei in der allgemeinen Formel (IIb)
• die Variable p eine ganze Zahl von 1 bis 4, vorzugsweise 1 oder 2, besonders bevorzugt 1, darstellt,
• der Rest R⁵ jeweils unabhängig voneinander, gleich oder verschieden, darstellt: Wasserstoff, einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃-CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃- CH(OR⁶) - CH₂ - C(O) - darstellt.

10. Gegebenenfalls C₅-C₃₄-fettsäurefunktionalisierter 3-Hydroxybuttersäure-Polyglycerinester (II) nach Anspruch 9,
wobei der gegebenenfalls C₅-C₃₄-fettsäurefunktionalisierte 3-Hydroxybuttersäure-Polyglycerinester (II) ein 3-Hydroxybuttersäure-Polyglycerinester der allgemeinen Formel (IIb")
R⁷O - CH₂ - CH(OR⁷) - CH₂ - [O - CH₂ - CH(OR⁷) - CH₂]ₚ - OR⁷ (IIb")
ist, wobei in der allgemeinen Formel (IIb")
• die Variable p eine ganze Zahl von 1 bis 4, vorzugsweise 1 oder 2, besonders bevorzugt 1, darstellt,
• der Rest R⁷ jeweils unabhängig voneinander, gleich oder verschieden, darstellt: Wasserstoff oder CH₃ - CH(OH) - CH₂ - C(O) -, jedoch mit der Maßgabe, dass mindestens ein Rest R¹, insbesondere mindestens zwei Reste R⁷, keinen Wasserstoff darstellt.

11. Gegebenenfalls C₅-C₃₄-fettsäurefunktionalisierter 3-Hydroxybuttersäure-Polyglycerinester (II) nach Anspruch 9,
wobei der gegebenenfalls C₅-C₃₄-fettsäurefunktionalisierte 3-Hydroxybuttersäure-Polyglycerinester (II) ein C₅-C₃₄-fettsäurefunktionalisierter, besonders bevorzugt C₈-C₃₄-fettsäurefunktionalisierter, 3-Hydroxybuttersäure-Polyglycerinester der allgemeinen Formel (IIb‴)
R⁵O - CH₂ - CH(OR⁵) - CH₂ - [O - CH₂ - CH(OR⁵) - CH₂]ₚ - OR⁵ (IIb‴)
ist, wobei in der allgemeinen Formel (IIb‴)
• die Variable p eine ganze Zahl von 1 bis 4, vorzugsweise 1 oder 2, besonders bevorzugt 1, darstellt,
• der Rest R⁵ jeweils unabhängig voneinander, gleich oder verschieden, darstellt: Wasserstoff, einen Rest R², wobei der Rest R² einen Rest vom Typ lineares oder verzweigtes, gesättigtes oder ein- oder mehrfach ungesättigtes (C₄-C₃₃-Alkyl) - C(O) -, bevorzugt (C₇-C₃₃-Alkyl) - C(O) -, darstellt, oder einen Rest CH₃-CH(OR⁶) - CH₂ - C(O) -, wobei der Rest R⁶ Wasserstoff oder einen Rest R², wie zuvor definiert, darstellt, jedoch mit der Maßgabe, dass mindestens ein Rest R⁵, insbesondere mindestens zwei Reste R⁵, keinen Wasserstoff darstellt, und mit der Maßgabe, dass mindestens ein Rest R⁵, insbesondere mindestens zwei Reste R⁵, einen Rest CH₃- CH(OR⁶) - CH₂ - C(O) - darstellt, und mit der Maßgabe, dass mindestens ein Rest R⁵ und/oder ein Rest R⁶ einen Rest R² darstellt.

12. Gemisch, enthaltend mindestens zwei, vorzugsweise mindestens drei, voneinander verschiedene, gegebenenfalls C₅-C₃₄-fettsäurefunktionalisierte, insbesondere gegebenenfalls C₈-C₃₄-fettsäurefunktionalisierte, 3-Hydroxybuttersäure-Polyglycerinester (II) gemäß einem der Ansprüche 9 bis 11.

13. Pharmazeutische Zusammensetzung, insbesondere Arzneimittel oder Medikament, umfassend ein Reaktionsprodukt gemäß Anspruch 8 und/oder einen oder mehrere C₅-C₃₄-fettsäurefunktionalisierte, besonders bevorzugt C₈-C₃₄-fettsäurefunktionalisierte, 3-Hydroxybuttersäure-Polyglycerinester gemäß einem der Ansprüche 9 bis 12.

14. Pharmazeutische Zusammensetzung nach Anspruch 13 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

15. Nahrungsmittel- und/oder Lebensmittelerzeugnis, umfassend ein Reaktionsprodukt gemäß Anspruch 8 und/oder ein oder mehrere C₅-C₃₄-fettsäurefunktionalisierte, besonders bevorzugt C₈-C₃₄-fettsäurefunktionalisierte, 3-Hydroxybuttersäure-Polyglycerinester gemäß einem der Ansprüche 9 bis 12.

## Claims

1. A method for producing a polyglycerol ester of 3-hydroxybutyric acid, which polyglycerol ester is optionally C₅-C₃₄-fatty acid functionalized, especially optionally C₈-C₃₄-fatty acid functionalized,
wherein an optionally C₅-C₃₄-fatty acid functionalized, especially optionally C₈-C₃₄-fatty acid functionalized, 3-oxobutyric acid polyglycerol ester (I) which comprises at least one 3-oxobutyrate radical (3-oxobutyric acid radical) of the general formula (I')
CH₃ - C(O) - CH₂ - C(O)O - (I')
is subjected, by means of at least one reducing agent, to a selective reduction of the at least one 3-oxobutyrate radical of the general formula (I') positioned at the keto group - C(O) - of the acetyl function CH₃ - C(O) - so as to result a 3-hydroxybutyrate radical (3-hydroxybutyric acid radical) of the general formula (II')
CH₃ - CH(OH) - CH₂ - C(O)O - (II')
wherein the optionally C₅-C₃₄-fatty acid functionalized, especially optionally C₈-C₃₄-fatty acid functionalized, 3-oxobutyric acid polyglycerol ester (I), used as a starting compound, corresponds to the general formula (Ib)
R¹O - CH₂ - CH(OR¹) - CH₂ - [O - CH₂ - CH(OR¹) - CH₂]ₚ - OR¹ (Ib)
wherein, in the general formula (Ib),
• the variable p represents an integer from 1 to 4, preferentially 1 or 2, more preferably 1,
• the radical R¹, each independently of one another, identical or different, represents: hydrogen, CH₃ - C(O) - CH₂ - C(O) - or a radical R², wherein the radical R² represents a radical of the type linear or branched, saturated or mono- or polyunsaturated (C₄-C₃₃-alkyl) - C(O) -, preferably (C₇-C₃₃-alkyl) - C(O) -, however, with the proviso that at least one radical R¹, especially at least two radicals R¹, does not represent hydrogen, and with the proviso that at least one radical R¹, especially at least two radicals R¹, represents a radical CH₃- C(O) - CH₂ - C(O) -; and
wherein the reducing agent is selected from the group of hydrogen, hydrides and alcohols as well as mixtures thereof; and
wherein the reduction is carried out as a complete reduction or else as a non-complete reduction of all keto groups - C(O) - of the acetyl function CH₃ - C(O) - of the at least one 3-oxobutyrate radical of the general formula (I') to a 3-hydroxybutyrate radical of the general formula (II');
so that, as a reaction product, an optionally C₅-C₃₄-fatty acid functionalized, especially optionally C₈-C₃₄-fatty acid functionalized, 3-hydroxybutyric acid polyglycerol ester (II) is obtained.

2. The method according to claim 1,
wherein the reducing agent is selected from the group of hydrogen, hydrides, especially inorganic hydrides, and alcohols, especially C₁-C₄-alcohols, as well as mixtures thereof, preferentially selected from the group of hydrogen, alkali metal or alkaline earth metal borohydride, alkali metal or alkaline earth metal hydride, alkali metal or alkaline earth metal aluminum hydride, methanol, ethanol, propanol and isopropanol as well as mixtures thereof, preferably from the group of hydrogen, alkali metal or alkaline earth metal borohydride and isopropanol.

3. The method according to claim 1 or claim 2,
wherein the reduction is carried out autocatalytically or in the presence of a catalyst;
especially wherein, in case of carrying out the reduction in the presence of a catalyst, the catalyst is recycled after the reduction has been carried out.

4. The method according to any of claims 1 to 3,
wherein the reduction is carried out in the presence of a catalyst;
especially wherein the catalyst is recycled after the reduction has been carried out; and/or
especially wherein the catalyst is selected from enzymes and/or metals or metal compounds, especially noble metals and transition metal compounds; and/or
especially wherein the catalyst is selected from the group of dehydrogenases, especially alcohol dehydrogenases, and metals and/or metal compounds based on palladium, platinum, rhodium, iridium, ruthenium and nickel, preferentially from the group of dehydrogenases, especially alcohol dehydrogenases, and metals and/or metal compounds based on platinum, palladium, nickel and rhodium.

5. The method according to any of claims 1 to 4,
wherein the reduction is carried out such that the optionally C₅-C₃₄-fatty acid functionalized, especially optionally C₈-C₃₄-fatty acid functionalized, 3-hydroxybutyric acid polyol ester (II), especially 3-hydroxybutyric acid polyglycerol ester, obtainable as a reaction product, does not comprise an acetyl function CH₃ - C(O) -; and/or
wherein the reducing agent is used in molar amounts, based on to the keto groups - C(O) - of the acetyl function CH₃ - C(O) - of the at least one 3-oxobutyrate radical of the general formula (I'), in a range of from equimolar amount to a molar excess of 200 mol-%, especially in a range of from equimolar amount to a molar excess of 150 mol-%, preferentially in a range of from equimolar amount to a molar excess of 100 mol-%; and/or
wherein the reducing agent on the one hand and the optionally C₅-C₃₄-fatty acid functionalized, especially optionally C₈-C₃₄-fatty acid functionalized, 3-oxobutyric acid polyol esters (I) to be reduced, on the other hand, are used in a molar ratio of reducing agent/keto groups - C(O) - of the acetyl function CH₃ - C(O) - of the 3-oxobutyrate radical of the general formula (I'), as defined hereinabove, in a range of from 1:1 bis 10 : 1, especially in a range of from 2 : 1 bis 8 : 1, preferentially in a range of from 3 : 1 bis 6 : 1.

6. The method according to any of claims 1 to 4,
wherein the reduction is carried out such that the reaction product comprises at least one acetyl function CH₃ - C(O) - and/or wherein the reduction is carried out such that the reaction product comprises at least one 3-oxobutyrate radical of the general formula (I'); and/or
wherein the reducing agent is used in molar amounts below the equimolar amount and/or in a substoichiometric amount relative to the keto groups - C(O) - of the acetyl function CH₃ - C(O) - of the at least one 3-oxobutyrate radical of the general formula (I').

7. The method according to any of the preceding claims,
wherein hydroxyl groups still present in the reaction product (II) after the reduction has been carried out are at least partially, preferentially completely, functionalized, especially esterified; and/or
wherein the reduction is followed by a partial, especially complete, functionalization, especially esterification, of hydroxyl groups still present; and/or
wherein ester groups present in the reaction product (II) after the reduction has been carried out, especially in the form of 3-hydroxybutyrate radicals according to the general formula (II') as defined hereinabove, are partially transesterified;
wherein the reduction is followed by a partial transesterification of ester groups present, especially in the form of 3-hydroxybutyrate radicals according to the general formula (II') as defined hereinabove;
especially wherein the functionalization and/or transesterification is carried out by means of a C₅-C₃₄-fatty acid, preferably a C₈-C₃₄-fatty acid, especially in free form or in the form of its ester or anhydride; and/or
especially wherein the C₅-C₃₄-fatty acid, preferably the C₈-C₃₄-fatty acid, especially in free form or in the form of its ester or anhydride, is selected from the group of caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachidic acid, heneicosanoic acid, behenic acid, lignoceric acid, cerotinic acid, montanic acid, melissic acid, laccic acid, geddic acid, undecylic acid, myristoleic acid, palmitoleic acid, margaroleic acid, petroselinic acid, oleic acid, elaidic acid, vaccenic acid, gadoleic acid, cetoleic acid, erucic acid, nervonic acid, linoleic acid, linolenic acids, calendulic acid, punicic acid, eleostearic acids, stearidonic acid, arachidonic acid, eicosapentaenoic acid, docosadienoic acid, docosatetraenoic acid, docosapentaenoic acid, docosahexaenoic acid, and tetracosahexaenoic acid, as well as mixtures thereof; and/or
especially wherein the C₅-C₃₄-fatty acid, preferably the C₈-C₃₄-fatty acid, especially in free form or in the form of its ester or anhydride, is selected from the group of myristic acid, pentadecanoic acid, palmitoleic acid, cetoleic acid, oleic acid, gadoleic acid,
cetoleic acid, erucic acid, arachidonic acid, eicosapentaenoic acid, docosadienoic acid, docosatetraenoic acid, docosapentaenoic acid, docosahexaenoic acid, tetracosahexaenoic acid as well as mixtures thereof, preferentially eicosapentaenoic acid and docosahexaenoic acid as well as mixtures thereof; and/or
especially wherein the C₅-C₃₄-fatty acid, preferably the C₈-C₃₄-fatty acid, especially in free form or in the form of its ester or anhydride, is selected from the group of fatty acids based on fish oil and/or occurring in fish oils, especially eicosapentaenoic acid, docosadienoic acid, docosatetraenoic acid, docosapentaenoic acid, docosahexaenoic acid and tetracosahexaenoic acid as well as mixtures thereof, preferentially eicosapentaenoic acid, docosahexaenoic acid as well as mixtures thereof.

8. A reaction product, namely optionally C₅-C₃₄-fatty acid functionalized, especially optionally C₈-C₃₄-fatty acid functionalized, 3-hydroxybutyric acid polyglycerol ester (II), obtainable according to the method of any of the preceding claims.

9. An optionally C₅-C₃₄-fatty acid functionalized, especially optionally C₈-C₃₄-fatty acid functionalized, 3-hydroxybutyric acid polyglycerol ester of the general formula (IIb)
R⁵O - CH₂ - CH(OR⁵) - CH₂ - [O - CH₂ - CH(OR⁵) - CH₂]ₚ - OR⁵ (IIb)
wherein, in the general formula (IIb),
• the variable p represents an integer from 1 to 4, preferentially 1 or 2, more preferably 1,
• the radical R⁵, each independently of one another, identical or different, represents: hydrogen, a radical R², wherein the radical R² represents a radical of the type linear or branched, saturated or mono- or polyunsaturated (C₄-C₃₃-alkyl) - C(O) -, preferably (C₇-C₃₃-alkyl) - C(O) -, or a radical CH₃- CH(OR⁶) - CH₂ - C(O) -, wherein the radical R⁶ represents hydrogen or a radical R², as defined hereinabove, however, with the proviso that at least one radical R⁵, especially at least two radicals R⁵, does not represent hydrogen, and with the proviso that at least one radical R⁵, especially at least two radicals R⁵, represents a radical CH₃- CH(OR⁶) - CH₂ - C(O) -.

10. The optionally C₅-C₃₄-fatty acid functionalized 3-hydroxybutyric acid polyglycerol ester (II) according to claim 9,
wherein the optionally C₅-C₃₄-fatty acid functionalized 3-hydroxybutyric acid polyglycerol ester (II) is a 3-hydroxybutyric acid polyglycerol ester of the general formula (IIb")
R⁷O - CH₂ - CH(OR⁷) - CH₂ - [O - CH₂ - CH(OR⁷) - CH₂]ₚ - OR⁷ (IIb")
wherein, in the general formula (IIb"),
• the variable p represents an integer from 1 to 4, preferentially 1 or 2, more preferably 1,
• the radical R⁷, each independently of one another, identical or different, represents: hydrogen or CH₃ - CH(OH) - CH₂ - C(O) -, however, with the proviso that at least one radical R¹, especially at least two radicals R⁷, does not represent hydrogen.

11. The optionally C₅-C₃₄-fatty acid functionalized 3-hydroxybutyric acid polyglycerol ester (II) according to claim 9,
wherein the optionally C₅-C₃₄-fatty acid functionalized 3-hydroxybutyric acid polyglycerol ester (II) is a C₅-C₃₄-fatty acid functionalized, more preferably C₈-C₃₄-fatty acid functionalized, 3-hydroxybutyric acid polyglycerol ester of the general formula (IIb‴)
R⁵O - CH₂ - CH(OR⁵) - CH₂ - [O - CH₂ - CH(OR⁵) - CH₂]ₚ - OR⁵ (IIb‴)
wherein, in the general formula (IIb"),
• the variable p represents an integer from 1 to 4, preferentially 1 or 2, more preferably 1,
• the radical R⁵, each independently of one another, identical or different, represents: hydrogen, a radical R², wherein the radical R² respresents a radical of the type linear or branched, saturated or mono- or polyunsaturated (C₄-C₃₃-alkyl) - C(O) -, preferably (C₇-C₃₃-alkyl) - C(O) -, or a radical CH₃- CH(OR⁶) - CH₂ - C(O) -, wherein the radical R⁶ represents hydrogen or a radical R², as defined hereinabove, however, with the proviso that at least one radical R⁵, especially at least two radicals R⁵, does not represent hydrogen, and with the proviso that at least one radical R⁵, especially at least two radicals R⁵, represents a radical CH₃- CH(OR⁶) - CH₂ - C(O) -, and with the proviso that at least one radical R⁵ and/or one radical R⁶ represents a radical R².

12. A mixture comprising at least two, preferentially at least three, different optionally C₅-C₃₄-fatty acid functionalized, especially optionally C₈-C₃₄-fatty acid functionalized, 3-hydroxybutyric acid polyglycerol esters (II) according to any of claims 9 to 11.

13. A pharmaceutical composition, especially a drug or medicament, comprising a reaction product according to claim 8 and/or one or more C₅-C₃₄-fatty acid functionalized, more preferably C₈-C₃₄-fatty acid functionalized, 3-hydroxybutyric acid polyglycerol esters according to any of claims 9 to 12.

14. The pharmaceutical composition according to claim 13 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

15. A food and/or a food product comprising a reaction product according to claim 8 and/or one or more C₅-C₃₄-fatty acid functionalized, more preferably C₈-C₃₄-fatty acid functionalized, 3-hydroxybutyric acid polyglycerol esters according to any of claims 9 to 12.

## Revendications

1. Procédé de préparation d'un ester de polyglycérol de l'acide 3-hydroxybutyrique, éventuellement fonctionnalisé par des acides gras C₅-C₃₄, en particulier éventuellement fonctionnalisé par des acides gras C₈-C₃₄,
où un ester de polyglycérol et d'acide 3-oxobutyrique (I), éventuellement fonctionnalisé par des acides gras en C₅-C₃₄, en particulier éventuellement fonctionnalisé par des acides gras en C₈-C₃₄, qui contient au moins un radical 3-oxobutyrate (radical d'acide 3-oxobutyrique) de formule générale (I')
CH₃ - C(O) - CH₂ - C(O)O - (I')
est soumise, à l'aide d'au moins un agent réducteur, d'une réduction sélective du au moins un radical 3-oxobutyrate de formule générale (I') sur le groupe cétonique - C(O) - de la fonction acétyle CH₃ - C(O) - en un radical 3hydroxybutyrate (radical acide 3-hydroxybutyrique) de formule générale (II')
CH₃ - CH(OH)- CH₂ - C(O)O - (II')
où l'ester de polyglycérol de l'acide 3-oxobutyrique (I) utilisé comme composé de départ, éventuellement fonctionnalisé par des acides gras en C₅-C₃₄, en particulier éventuellement fonctionnalisé par des acides gras en C₈-C₃₄, correspond à la formule générale (Ib)
R¹ O - CH₂ - CH(OR¹) - CH₂ - [O - CH₂ - CH(OR¹) - CH₂]ₚ - OR¹ (Ib)
où, dans la formule générale (Ib),
• la variable p représente un nombre entier de 1 à 4, de préférence 1 ou 2, de manière particulièrement préférée 1,
• le radical R¹ représente chacun indépendamment, de manière identique ou différente : l'hydrogène, CH₃ - C(O) - CH₂ - C(O) - ou un radical R², le radical R² représentant un radical du type linéaire ou ramifié, saturé ou mono ou polyinsaturé (C₄-C₃₃-Alkyle) - C(O) -, de préférence (C₇-C₃₃-Alkyle) - C(O) -, à condition toutefois qu'au moins un radical R¹, en particulier au moins deux radicaux R¹, ne représente pas un hydrogène, et à condition qu'au moins un radical R¹, en particulier au moins deux radicaux R¹, représente un radical CH₃- C(O) - CH₂ - C(O) -; et
où l'agent réducteur est choisi parmi le groupe constitué par l'hydrogène, les hydrures et les alcools ainsi que leurs mélanges; et
où la réduction est effectuée comme une réduction complète ou bien comme une réduction non-complète de tous les groupes cétoniques - C(O) - de la fonction acétyle CH₃ - C(O) - du au moins un radical 3-oxobutyrate de formule générale (I') en un radical 3-hydroxybutyrate de formule générale (II');
de sorte que l'on obtient, comme produit de réaction, un ester de polyglycérol et d'acide 3-hydroxybutyrique (II), éventuellement fonctionnalisé par C₅-C₃₄-acide gras, en particulier éventuellement fonctionnalisé par C₈-C₃₄-acide gras.

2. Procédé selon la revendication 1,
où l'agent réducteur est choisi parmi le groupe constitué par l'hydrogène, les hydrures, en particulier les hydrures inorganiques, et les alcools, en particulier les alcools en C₁-C₄, ainsi que leurs mélanges, de préférence choisis parmi le groupe constitué par l'hydrogène, borohydrure alcalin ou alcalino-terreux, hydrure alcalin ou alcalino-terreux, hydrure alcalin ou alcalino-terreux d'aluminium, méthanol, éthanol, propanol et isopropanol ainsi que leurs mélanges, de préférence parmi le groupe constitué par l'hydrogène, le borohydrure alcalin ou alcalino-terreux et l'isopropanol.

3. Procédé selon la revendication 1 ou la revendication 2,
où la réduction est effectuée de manière autocatalytique ou en présence d'un catalyseur;
en particulier où, dans le cas où la réduction est effectuée en présence d'un catalyseur, le catalyseur est recyclé après que la réduction a été effectuée.

4. Procédé selon l'une quelconque des revendications 1 à 3,
où la réduction est effectuée en présence d'un catalyseur;
en particulier où le catalyseur est recyclé une fois la réduction effectuée; et/ou
en particulier où le catalyseur est choisi parmi des enzymes et/ou des métaux ou des composés métalliques, en particulier des métaux nobles et des composés de métaux de transition; et/ou
en particulier où le catalyseur est choisi parmi le groupe des déshydrogénases, en particulier des alcools déshydrogénases, et des métaux et/ou des composés métalliques à base de palladium, platine, rhodium, iridium, ruthénium et nickel, de préférence parmi le groupe des déshydrogénases, en particulier des alcools déshydrogénases, et des métaux et/ou des composés métalliques à base de platine, palladium, nickel et rhodium.

5. Procédé selon l'une quelconque des revendications 1 à 4,
où la réduction est effectuée de telle sorte que le polyolester (II) de l'acide 3-hydroxybutyrique, en particulier l'ester de polyglycérol de l'acide 3-hydroxybutyrique, éventuellement fonctionnalisé par un acide gras C₅-C₃₄, en particulier éventuellement fonctionnalisé par un acide gras C₈-C₃₄, obtenu comme produit de réaction ne présente pas de fonction acétyle CH₃ - C(O) -; et/ou
où l'agent réducteur, par rapport aux groupes cétoniques - C(O) - de la fonction acétyle CH₃ - C(O) - du au moins un radical 3-oxobutyrate de formule générale (I'), est présent en quantités molaires dans une plage allant d'une quantité équimolaire à un excès molaire de 200 % en moles, en particulier dans une plage allant d'une quantité équimolaire jusqu'à un excès molaire de 150 % en moles, de préférence dans une plage allant d'une quantité équimolaire jusqu'à un excès molaire de 100 % en moles; et/ou
où l'agent réducteur, d'une part, et le polyester d'acide 3-oxobutyrique (I) à réduire, éventuellement fonctionnalisé par des acides gras C₅-C₃₄, en particulier éventuellement fonctionnalisé par des acides gras C₈-C₃₄, d'autre part, sont utilisés dans un rapport molaire agent réducteur/groupes cétoniques -C(O)- de la fonction acétyle CH₃ - C(O) - du radical 3-oxobutyrate de formule générale (I'), telle que définie précédemment, dans une plage de 1 : 1 à 10 : 1, en particulier dans une plage de 2 : 1 à 8 : 1, de préférence dans une plage de 3 : 1 à 6 : 1.

6. Procédé selon l'une quelconque des revendications 1 à 4,
où la réduction est effectuée de telle sorte que le produit de réaction présente au moins une fonction acétyle CH₃ - C(O) - et/ou où la réduction est effectuée de telle sorte que le produit de réaction présente au moins un radical 3-oxobutyrate de formule générale (I'); et/ou
où l'agent réducteur, par rapport aux groupes cétoniques - C(O) - de la fonction acétyle CH₃ - C(O) - du au moins un radical 3-oxobutyrate de formule générale (I'), est utilisé en quantités molaires inférieures à la quantité équimolaire et/ou en quantité sous-stoechiométrique.

7. Procédé selon l'une quelconque des revendications précédentes,
où les groupes hydroxyle encore présents dans le produit de réaction (II) après la réduction sont au moins partiellement, de préférence complètement, fonctionnalisés, en particulier estérifiés; et/ou
où la réduction est suivie d'une fonctionnalisation partielle, en particulier complète, en particulier d'une estérification, des groupes hydroxyle encore présents; et/ou
où les groupes esters présents dans le produit de réaction (II) sont partiellement transestérifiés après la réduction, en particulier sous forme de radicaux 3-hydroxybutyrate selon la formule générale (II'), comme défini précédemment;
où la réduction est suivie d'une transestérification partielle des groupes esters présents, en particulier sous forme de radicaux 3-hydroxybutyrate selon la formule générale (II'), telle que définie précédemment;
en particulier où la fonctionnalisation et/ou la transestérification est effectuée au moyen d'un acide gras C₅-C₃₄, de préférence d'un acide gras C₈-C₃₄, en particulier sous forme libre ou sous forme de son ester ou anhydride; et/ou
en particulier où l'acide gras C₅-C₃₄, de préférence l'acide gras C₈-C₃₄, en particulier sous forme libre ou sous forme de son ester ou anhydride, est choisi parmi le groupe de l'acide caprylique, de l'acide pélargonique, de l'acide caprique, de l'acide undécanoïque, acide laurique, acide tridécanoïque, acide myristique, acide pentadécanoïque, acide palmitique, acide margarique, acide stéarique, acide nonadécanoïque, acide arachidique, acide hénéicosanoïque, acide béhénique, acide lignocérique, acide cérotique, acide montanique, acide mélissique, acide lacérique, acide geddique, acide undécylique, acide myristoléique, acide palmitoléique, acide margaroléique, acide pétrosélinique, acide oléique, acide élaïdique, acide vaccénique, acide gadoléique, acide cétoléique, acide érucique, acide nervonique, acide linoléique, acides linoléniques, l'acide calendulique, l'acide punicique, les acides éléostéariques, l'acide stéaridonique, l'acide arachidonique, l'acide eicosapentaénoïque, l'acide docosadiénoïque, l'acide docosatétraénoïque, l'acide docosapentaénoïque, l'acide docosahexaénoïque, et l'acide tétracosahexaénoïque, ainsi que leurs mélanges; et/ou
en particulier où l'acide gras C₅-C₃₄, de préférence l'acide gras C₈-C₃₄, en particulier sous forme libre ou sous forme de son ester ou anhydride, est choisi parmi le groupe de l'acide myristique, de l'acide pentadécanoïque, de l'acide palmitoléique, de l'acide cétoléique, de l'acide oléique, de l'acide gadoléique, acide cétoléique, acide érucique, acide arachidonique, acide eicosapentaénoïque, acide docosadiénoïque, acide docosatétraénoïque, acide docosapentaénoïque, acide docosahexaénoïque, acide tétracosahexaénoïque ainsi que leurs mélanges, de préférence l'acide eicosapentaénoïque et l'acide docosahexaénoïque ainsi que leurs mélanges; et/ou
en particulier où l'acide gras C₅-C₃₄, de préférence l'acide gras C₈-C₃₄, en particulier sous forme libre ou sous forme de son ester ou anhydride, est choisi parmi le groupe des acides gras à base d'huile de poisson et/ou présents dans les huiles de poisson, en particulier l'acide eicosapentaénoïque, l'acide docosadiénoïque, l'acide docosatétraénoïque, l'acide docosapentaénoïque, l'acide docosahexaénoïque et l'acide tétracosahexaénoïque ainsi que leurs mélanges, de préférence l'acide eicosapentaénoïque, l'acide docosahexaénoïque ainsi que leurs mélanges.

8. Produit de réaction, à savoir un ester de 3-hydroxybutyrate de polyglycérol (II), éventuellement fonctionnalisé par un acide gras C₅-C₃₄, en particulier éventuellement fonctionnalisé par un acide gras C₈-C₃₄, que l'on peut obtenir selon le procédé selon l'une des revendications précédentes.

9. Ester de polyglycérol de l'acide 3-hydroxybutyrique, éventuellement fonctionnalisé par des acides gras C₅-C₃₄, en particulier éventuellement fonctionnalisé par des acides gras C₈-C₃₄, de formule générale (IIb)
R⁵O - CH₂ - CH(OR⁵) - CH₂ - [O - CH₂ - CH(OR⁵) - CH₂]ₚ - OR⁵ (IIb)
où, dans la formule générale (IIb)
• la variable p représente un nombre entier de 1 à 4, de préférence 1 ou 2, de manière particulièrement préférée 1,
• le radical R⁵ représente chacun indépendamment l'un de l'autre, de manière identique ou différente : l'hydrogène, un radical R², le radical R² représentant un radical de type linéaire ou ramifié, saturé ou mono ou polyinsaturé (C₄-C₃₃-Alkyle) - C(O) -, de préférence (C₇-C₃₃-Alkyle) - C(O) -, ou un radical CH₃ - CH(OR⁶) - CH₂ - C(O) -, le radical R⁶ représentant l'hydrogène ou un radical R², tel que défini précédemment, à condition toutefois qu'au moins un radical R⁵, notamment au moins deux radicaux R⁵, ne représente pas un atome d'hydrogène, et à condition qu'au moins un radical R⁵, notamment au moins deux radicaux R⁵, représente un radical CH₃- CH(OR⁶) - CH₂ - C(O) -.

10. Ester de polyglycérol de l'acide 3-hydroxybutyrique. éventuellement fonctionnalisé par des acides gras C₅-C₃₄ (II), selon la revendication 9,
où l'ester polyglycérique de l'acide 3-hydroxybutyrique, éventuellement fonctionnalisé par des acides gras en C₅-C₃₄ (II), est un ester polyglycérique de l'acide 3-hydroxybutyrique de formule générale (IIb") R
⁷O - CH₂ - CH(OR⁷) - CH₂ - [O - CH₂ - CH(OR⁷) - CH₂]ₚ - OR⁷ (IIb")
où, dans la formule générale (IIb"),
• la variable p représente un nombre entier de 1 à 4, de préférence 1 ou 2, de manière particulièrement préférée 1,
• le reste R⁷ représente chaque fois indépendamment l'un de l'autre, de manière identique ou différente : l'hydrogène ou CH₃ - CH(OH) - CH₂ - C(O) --, à condition toutefois qu'au moins un radical R⁷, en particulier au moins deux radicaux R⁷, ne représente pas l'hydrogène.

11. Ester de polyglycérol de l'acide 3-hydroxybutyrique, éventuellement fonctionnalisé par des acides gras C₅-C₃₄ (II), selon la revendication 9,
où l'ester de polyglycérol de l'acide 3-hydroxybutyrique (II), éventuellement fonctionnalisé par des acides gras en C₅-C₃₄, est un ester de polyglycérol de l'acide 3-hydroxybutyrique fonctionnalisé par des acides gras en C₅-C₃₄, de préférence encore par des acides gras en C₈-C₃₄, de formule générale (IIb‴)
R⁵O - CH₂ - CH(OR⁵) - CH₂ - [O - CH₂ - CH(OR⁵) - CH₂]ₚ - OR⁵ (IIb‴)
où, dans la formule générale (IIb‴),
• la variable p représente un nombre entier de 1 à 4, de préférence 1 ou 2, de manière particulièrement préférée 1,
• le radical R⁵ représente chacun indépendamment l'un de l'autre, de manière identique ou différente : l'hydrogène, un radical R², le radical R² représentant un radical de type linéaire ou ramifié, saturé ou mono ou polyinsaturé (C₄-C₃₃-Alkyle) - C(O) -, de préférence (C₇-C₃₃-Alkyle) - C(O) -, ou un radical CH₃ - CH(OR⁶) - CH₂ - C(O) -, le radical R⁶ représentant l'hydrogène ou un radical R², tel que défini précédemment, à condition toutefois qu'au moins un radical R⁵, en particulier au moins deux radicaux R⁵, ne représente pas un atome d'hydrogène, et à condition qu'au moins un radical R⁵, en particulier au moins deux radicaux R⁵, représente un radical CH₃- CH(OR⁶) - CH₂ - C(O) -, et à condition qu'au moins un radical R⁵ et/ou un radical R⁶ représente un radical R².

12. Mélange contenant au moins deux, de préférence au moins trois, esters de polyglycérol et d'acide 3-hydroxybutyrique (II) différents les uns des autres, éventuellement fonctionnalisés par des acides gras en C₅-C₃₄, en particulier éventuellement fonctionnalisés par des acides gras en C₈-C₃₄ , selon l'une des revendications 9 à 11.

13. Composition pharmaceutique, en particulier médicament ou médicament, comprenant un produit de réaction selon la revendication 8 et/ou un ou plusieurs esters de polyglycérol et d'acide 3-hydroxybutyrique fonctionnalisés par des acides gras en C₅-C₃₄, de préférence encore fonctionnalisés par des acides gras en C₈-C₃₄, selon l'une quelconque des revendications 9 à 12.

14. Composition pharmaceutique selon la revendication 13 pour son utilisation dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

15. Produit alimentaire et/ou nutritionnel comprenant un produit de réaction selon la revendication 8 et/ou un ou plusieurs esters de polyglycérol et d'acide 3-hydroxybutyrique fonctionnalisés par des acides gras en C₅-C₃₄ , de préférence encore fonctionnalisés par des acides gras en C₈-C₃₄, selon l'une quelconque des revendications 9 à 12.
